# EUROPEAN PATENT SPECIFICATION

(11) **EP 3 274 448 B1**
(45) Date of publication and mention of the grant of the patent: **11.08.2021**
(21) Application number: 16711641.7
(22) Date of filing: 23.03.2016
(51) Int. Cl.: C12N 9/10, C12P 19/56, C12N 9/02, A23L 2/60, A23L 27/30

(54) **UDP-GLYCOSYLTRANSFERASES FROM SOLANUM LYCOPERSICUM**
UDP-GLYCOSYLTRANSFERASEN AUS SOLANUM LYCOPERSICUM
UDP-GLYCOSYLTRANSFÉRASES DE SOLANUM LYCOPERSICUM

(30) Priority: 23.03.2015 US 201562136759 P
(43) Date of publication of application: 31.01.2018
(73) Proprietor: DSM IP Assets B.V., 6411 TE Heerlen (NL)
(72) Inventor: BOSCH, Hendrik, Jan, 6700 AA Wageningen (NL); BEEKWILDER, Martinus, Julius, 6700 AA Wageningen (NL); BOER, Viktor, Marius, 6100 AA Echt (NL)
(74) Representative: DSM Intellectual Property
(86) International application number: PCT/EP2016/056449
(87) International publication number: WO 2016/151046

(56) References cited:
- WO-A1-2013/110673
- WO-A1-2014/191580
- WO-A1-2014/193934
- WO-A1-2014/195944
- WO-A1-2015/014969
- WO-A2-2013/022989
- WO-A2-2014/191581
- DATABASE NCBI [Online] NIH.GOV; 19 November 2014 (2014-11-19), "crocetin glucoside glucosyltransferase-like [Solanum lycopersicum]", XP002757369, Database accession no. XP_004249995
- DATABASE UniProt [Online] 4 February 2015 (2015-02-04), "RecName: Full=Glycosyltransferase {ECO:0000256|RuleBase:RU362057}; EC=2.4.1.- {ECO:0000256|RuleBase:RU362057};", XP002757370, retrieved from EBI accession no. UNIPROT:K4D509 Database accession no. K4D509 -& DATABASE UniProt [Online] 22 July 2015 (2015-07-22), "RecName: Full=Glycosyltransferase {ECO:0000256|RuleBase:RU362057}; EC=2.4.1.- {ECO:0000256|RuleBase:RU362057};", XP002757371, retrieved from EBI accession no. UNIPROT:K4D509 Database accession no. K4D509 & WANG Y ET AL: "Characteristics of the tomato nuclear genome as determined by sequencing undermethylated EcoRI digested fragments", THEORETICAL AND APPLIED GENETICS ; INTERNATIONAL JOURNAL OF PLANT BREEDING RESEARCH, SPRINGER, BERLIN, DE, vol. 112, no. 1, 1 December 2005 (2005-12-01), pages 72-84, XP019322118, ISSN: 1432-2242, DOI: 10.1007/S00122-005-0107-Z
- THOMAS LOUVEAU ET AL: "Predicting the substrate specificity of a glycosyltransferase implicated in the production of phenolic volatiles in tomato fruit", FEBS JOURNAL, vol. 278, no. 2, 17 January 2011 (2011-01-17), pages 390-400, XP055247672, GB ISSN: 1742-464X, DOI: 10.1111/j.1742-4658.2010.07962.x
- DATABASE Geneseq [Online] 12 September 2013 (2013-09-12), "Stevia rebaudiana UGT2 gene, SEQ: 87.", XP002757289, retrieved from EBI accession no. GSN:BAR69149 Database accession no. BAR69149
- DATABASE Geneseq [Online] 12 September 2013 (2013-09-12), "Stevia rebaudiana UGT2 protein, SEQ: 88.", XP002757288, retrieved from EBI accession no. GSP:BAR69150 Database accession no. BAR69150
- DATABASE Geneseq [Online] 29 January 2015 (2015-01-29), "S. rebaudiana UDP-glycosyltransferase (UGT) protein (UGT2_1b), SEQ 100.", XP002757291, retrieved from EBI accession no. GSP:BBQ97935 Database accession no. BBQ97935
- DATABASE Geneseq [Online] 29 January 2015 (2015-01-29), "Stevia rebaudiana UDP-glycosyltransferase (UGT) protein (UGT2_1a) SEQ 88.", XP002757290, retrieved from EBI accession no. GSP:BBQ97923 Database accession no. BBQ97923
- DATABASE Geneseq [Online] 26 March 2015 (2015-03-26), "Stevia rebaudiana UDP-glycosyltransferase (UGT) protein, SEQ ID 100.", XP002757292, retrieved from EBI accession no. GSP:BBU39053 Database accession no. BBU39053
- DATABASE Geneseq [Online] 29 January 2015 (2015-01-29), "Stevia rebaudiana UGT2 protein, SEQ: 88.", XP002757293, retrieved from EBI accession no. GSP:BBR03844 Database accession no. BBR03844
- DATABASE Geneseq [Online] 11 April 2013 (2013-04-11), "Stevia rebaudiana UGT 91d2e polypeptide, SEQ ID:5.", XP002757294, retrieved from EBI accession no. GSP:BAK52046 Database accession no. BAK52046
- DATABASE UniProt [Online] 28 November 2012 (2012-11-28), "RecName: Full=Glycosyltransferase {ECO:0000256|RuleBase:RU362057}; EC=2.4.1.- {ECO:0000256|RuleBase:RU362057};", XP002760244, retrieved from EBI accession no. UNIPROT:K4BWC0 Database accession no. K4BWC0 -& M A Budiman ET AL: "A deep-coverage tomato BAC library and prospects toward development of an STC framework for genome sequencing", Genome research, 1 January 2000 (2000-01-01), pages 129-136, XP055190942, UNITED STATES Retrieved from the Internet: URL:http://www.ncbi.nlm.nih.gov/pubmed/106 45957
- PRAVEEN GULERIA ET AL: "Agrobacterium Mediated Transient Gene Silencing (AMTS) in Stevia rebaudiana: Insights into Steviol Glycoside Biosynthesis Pathway", PLOS ONE, vol. 8, no. 9, 4 September 2013 (2013-09-04), page e74731, XP055269932, DOI: 10.1371/journal.pone.0074731
- PRAVEEN GULERIA ET AL: "Insights into Steviol Glycoside Biosynthesis Pathway Enzymes Through Structural Homology Modeling", AMERICAN JOURNAL OF BIOCHEMISTRY AND MOLECULAR BIOLOGY, vol. 3, no. 1, 4 October 2012 (2012-10-04) , pages 1-19, XP055270235, ISSN: 2150-4210, DOI: 10.3923/ajbmb.2013.1.19

## Description

### Field of the invention

The invention relates to a recombinant host comprising a recombinant nucleic acid sequence encoding a UDP-glycosyltransferase (UGT) polypeptide. The invention also relates to a process for the preparation of a glycosylated diterpene using such a recombinant host and to a fermentation broth which may be the result of such a process. The invention further relates to a glycosylated diterpene obtained by such a process or obtainable from such a fermentation broth and to a composition comprising two or more such glycosylated diterpenes. In addition the invention relates to a foodstuff, feed or beverage which comprises such a glycosylated diterpene or a such composition. The invention also relates to a method for converting a first glycosylated diterpene into a second glycosylated diterpene using the above-mentioned recombinant host.

### Background to the invention

The leaves of the perennial herb, *Stevia rebaudiana* Bert., accumulate quantities of intensely sweet compounds known as steviol glycosides. Whilst the biological function of these compounds is unclear, they have commercial significance as alternative high potency sweeteners.

These sweet steviol glycosides have functional and sensory properties that appear to be superior to those of many high potency sweeteners. In addition, studies suggest that stevioside can reduce blood glucose levels in Type II diabetics and can reduce blood pressure in mildly hypertensive patients.

Steviol glycosides accumulate in Stevia leaves where they may comprise from 10 to 20% of the leaf dry weight. Stevioside and rebaudioside A are both heat and pH stable and suitable for use in carbonated beverages and many other foods. Stevioside is between 110 and 270 times sweeter than sucrose, rebaudioside A between 150 and 320 times sweeter than sucrose. In addition, rebaudioside D is also a high-potency diterpene glycoside sweetener which accumulates in Stevia leaves. It may be about 200 times sweeter than sucrose. Rebaudioside M is a further high-potency diterpene glycoside sweetener. It is present in trace amounts in certain stevia variety leaves, but has been suggested to have a superior taste profile.

Steviol glycosides have traditionally been extracted from the *Stevia* plant. In *Stevia,* (-)-kaurenoic acid, an intermediate in gibberellic acid (GA) biosynthesis, is converted into the tetracyclic diterpene steviol, which then proceeds through a multi-step glycosylation pathway to form the various steviol glycosides. However, yields may be variable and affected by agriculture and environmental conditions. Also, Stevia cultivation requires substantial land area, a long time prior to harvest, intensive labour and additional costs for the extraction and purification of the glycosides.

More recently, interest has grown in producing steviol glycosides using fermentative processes. WO2013/110673 and WO2015/007748 describe microorganisms that may be used to produce at least the steviol glycosides rebaudioside A and rebaudioside D. WO2014/193934, WO 2014/191580, WO2015/014969, WO2014/191581 and WO2013/022989 also relate to a recombinant microorganism for the production of steviol glycosides.

Further improvement of such microorganisms is desirable in order that higher amounts of steviol glycosides may be produced and/or additional or new steviol glycosides and/or higher amounts of specific steviol glycosides and/or mixtures of steviol glycosides having desired ratios of different steviol glycosides.

### Summary of the invention

The invention is represented by the claims herein.

In *Stevia rebaudiana,* steviol is synthesized from GGPP, which is formed by the deoxyxylulose 5- phosphate pathway. The activity of two diterpene cyclases (-)-copalyl diphosphate synthase (CPS) and (-)-kaurene synthase (KS) results in the formation of (-)-Kaurene which is then oxidized in a three step reaction by (-)-kaurene oxidase (KO) to form (-)-kaurenoic acid.

In *Stevia rebaudiana* leaves, (-)-kaurenoic acid is then hydroxylated, by ent-kaurenoic acid 13-hydroxylase (KAH) to form steviol. Steviol is then glycosylated by a series of UDP-glycosyltransferases (UGTs) leading to the formation of a number of steviol glycosides. Specifically, these molecules can be viewed as a steviol molecule, with its carboxyl hydrogen atom replaced by a glucose molecule to form an ester, and an hydroxyl hydrogen with combinations of glucose and rhamnose to form an acetal.

These pathways may be reconstructed in recombinant hosts, for example yeasts such as yeasts of the genera *Saccharomyces* and *Yarrowia.*

Disclosed is the identification of polypeptides having UDP-glycosyltransferase (UGT), typically having improved properties in comparison to those that are currently known. These polypeptides may be used to generate recombinant hosts that produce higher amounts of steviol glycosides and/or additional or new steviol glycosides and/or higher amounts of specific steviol glycosides and/or mixtures of steviol glycosides having desired ratios of different steviol glycosides.

Also disclosed is a recombinant host capable of producing a glycosylated diterpene, i.e. a diterpene glycoside such as a steviol glycoside, for example steviolmonoside, steviolbioside, stevioside, rebaudioside A, rebaudioside B, rebaudioside C, rebaudioside D, rebaudioside E, rebaudioside F, rebaudioside M, rubusoside, dulcoside A, steviol-13-monoside, steviol-19-monoside or 13-[(β-D-Glucopyranosyl)oxy)kaur-16-en-18-oic acid 2-O-β-D-glucopyranosyl-β-D-glucopyranosyl ester steviol-19-diside.

Accordingly, disclosed is a recombinant host comprising a recombinant nucleic acid sequence, typically having UDP-glycosyltransferase (UGT) activity such as UGT2 activity, encoding a polypeptide having:
a. the amino acid sequence set forth in SEQ ID NO: 1 or an amino acid sequence having at least about 30% sequence identity thereto;
b. the amino acid sequence set forth in SEQ ID NO: 2 or an amino acid sequence having at least about 30% sequence identity thereto;
c. the amino acid sequence set forth in SEQ ID NO: 3 or an amino acid sequence having at least about 30% sequence identity thereto; or
d. the amino acid sequence set forth in SEQ ID NO: 4 or an amino acid sequence having at least about 30% sequence identity thereto.

Also disclosed is:
- a process for the preparation of a glycosylated diterpene which comprises fermenting a disclosed recombinant host in a suitable fermentation medium, and optionally recovering the glycosylated diterpene;
- a fermentation broth comprising a glycosylated diterpene obtainable by the disclosed process;
- a glycosylated diterpene obtained by such a process or obtainable from such a fermentation broth;
- a composition comprising two or more such diterpenes;
- a foodstuff, feed or beverage which comprises such a glycosylated diterpene; and
- a method for converting a first glycosylated diterpene into a second glycosylated diterpene, which method comprises:
   - contacting said first glycosylated diterpene with a disclosed recombinant host, a cell free extract derived from such a recombinant host or an enzyme preparation derived from either thereof;
   - thereby to convert the first glycosylated diterpene into the second glycosylated diterpene.

### Brief description of the drawings

Figure 1 sets out Western blot detection of His-tagged UGTs
Figure 2 sets out Western blot of UGT2_1a and RT18. Lanes 1,2,3,4: 0.5, 1.0, 1.9, 3.8 µg of UGT2_1a crude enzyme extract. Lane 5 and 6: 31.9 and 63.8 µg RT18 crude enzyme extract.
Figure 3 sets out the effect of the expression of RT18 on the production of RebM
Figure 4 sets out the effect of the expression of RT18 on the production of RebD
Figure 5 sets out a schematic diagram of the potential pathways leading to biosynthesis of steviol glycosides.
Figure 6 sets out a schematic diagram of the potential pathways leading to biosynthesis of steviol glycosides. The compound shown with an asterisk is 13-[(β-D-Glucopyranosyl)oxy)kaur-16-en-18-oic acid 2-O-β-D-glucopyranosyl-β-D-glucopyranosyl ester.

### Description of the sequence listing

A description of the sequences is set out in Table 10. Sequences described herein may be defined with reference to the sequence listing or with reference to the database accession numbers also set out herein, for example in Table 10.

### Detailed description of the invention

Throughout the present specification and the accompanying claims, the words "comprise", "include" and "having" and variations such as "comprises", "comprising", "includes" and "including" are to be interpreted inclusively. That is, these words are intended to convey the possible inclusion of other elements or integers not specifically recited, where the context allows.

The articles "a" and "an" are used herein to refer to one or to more than one (i.e. to one or at least one) of the grammatical object of the article. By way of example, "an element" may mean one element or more than one element.

Herein, "rebaudioside" may be shortened to "reb". That is to say, rebaudioside A and reb A, for example, are intended to indicate the same molecule.

The term "recombinant" when used in reference to a cell, nucleic acid, protein or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all. The term "recombinant" is synonymous with "genetically modified".

Disclosed are polypeptides identified as having UDP-glycosyltransferase (UGT) activity which can be used in recombinant hosts, typically for the production of diterpene glycosides, such as steviol glycosides.

Herein, a polypeptide having UGT activity is one which has glycosyltransferase activity (EC 2.4), i.e. that can act as a catalyst for the transfer of a monosaccharide unit from an activated nucleotide sugar (also known as the "glycosyl donor") to a glycosyl acceptor molecule, usually an alcohol. The glycosyl donor for a UGT is typically the nucleotide sugar uridine diphosphate glucose (uracil-diphosphate glucose, UDP-glucose). A polypeptide suitable for use in a disclosed host typically has UGT activity and a polynucleotide sequence as described herein typically encodes such a polypeptide. Typically, the polypeptides for use in a disclosed host are polypeptides having UGT2-type activity.

Disclosed is thus a recombinant host comprising a recombinant nucleic acid sequence encoding a polypeptide comprising:
a. the amino acid sequence set forth in SEQ ID NO: 1 or an amino acid sequence having at least about 30% sequence identity thereto;
b. the amino acid sequence set forth in SEQ ID NO: 2 or an amino acid sequence having at least about 30% sequence identity thereto;
c. the amino acid sequence set forth in SEQ ID NO: 3 or an amino acid sequence having at least about 30% sequence identity thereto; or
d. the amino acid sequence set forth in SEQ ID NO: 4 or an amino acid sequence having at least about 30% sequence identity thereto.

The polypeptide encoded by the recombinant nucleic acid sequence typically has UGT activity, such as UGT2 activity. A disclosed recombinant host is typically capable of producing a glycosylated diterpene, for example a steviol glycoside.

A polypeptide encoded by a recombinant nucleic acid present in a disclosed recombinant host may comprise an amino acid sequence having at least about 35%, at least about 40%, at least about 50%, at least about 60%, at least about 65%, at least about 70%, at least about 75%, at least about 80%, at least about 85%, at least about, 86%, at least about 87%, at least about 88%, at least about 89%, at least about 90%, at least about 91%, at least about 92%, at least about 93%, at least about 94%, at least about 95%, at least about 96%, at least about 97%, at least about 98% or at least about 99% sequence identity to any one of SEQ ID NOs: 1, 2, 3 or 4.

Disclosed is:
- a recombinant host comprising a recombinant nucleic acid sequence encoding a polypeptide, typically having UGT activity, which comprises an amino acid sequence having at least about 35%, at least about 40%, at least about 50%, at least about 60%, at least about 65%, at least about 70%, at least about 75%, at least about 80%, at least about 85%, at least about, 86%, at least about 87%, at least about 88%, at least about 89%, at least about 90%, at least about 91%, at least about 92%, at least about 93%, at least about 94%, at least about 95%, at least about 96%, at least about 97%, at least about 98% or at least about 99% sequence identity to SEQ ID NO: 1;
- a recombinant host comprising a recombinant nucleic acid sequence encoding a polypeptide, typically having UGT activity, which comprises an amino acid sequence having at least about 35%, at least about 40%, at least about 50%, at least about 60%, at least about 65%, at least about 70%, at least about 75%, at least about 80%, at least about 85%, at least about, 86%, at least about 87%, at least about 88%, at least about 89%, at least about 90%, at least about 91%, at least about 92%, at least about 93%, at least about 94%, at least about 95%, at least about 96%, at least about 97%, at least about 98% or at least about 99% sequence identity to SEQ ID NO: 2;
- a recombinant host comprising a recombinant nucleic acid sequence encoding a polypeptide, typically having UGT activity, which comprises an amino acid sequence having at least about 35%, at least about 40%, at least about 50%, at least about 60%, at least about 65%, at least about 70%, at least about 75%, at least about 80%, at least about 85%, at least about, 86%, at least about 87%, at least about 88%, at least about 89%, at least about 90%, at least about 91%, at least about 92%, at least about 93%, at least about 94%, at least about 95%, at least about 96%, at least about 97%, at least about 98% or at least about 99% sequence identity to SEQ ID NO: 3;
- a recombinant host comprising a recombinant nucleic acid sequence encoding a polypeptide, typically having UGT activity, which comprises an amino acid sequence having at least about 35%, at least about 40%, at least about 50%, at least about 60%, at least about 65%, at least about 70%, at least about 75%, at least about 80%, at least about 85%, at least about, 86%, at least about 87%, at least about 88%, at least about 89%, at least about 90%, at least about 91%, at least about 92%, at least about 93%, at least about 94%, at least about 95%, at least about 96%, at least about 97%, at least about 98% or at least about 99% sequence identity to SEQ ID NO: 4.

As used herein, the term "polypeptide" refers to a molecule comprising amino acid residues linked by peptide bonds and containing more than five amino acid residues. The amino acids are identified by either the single-letter or three-letter designations. The term "protein" as used herein is synonymous with the term "polypeptide" and may also refer to two or more polypeptides. Thus, the terms "protein", "peptide" and "polypeptide" can be used interchangeably. Polypeptides may optionally be modified (e.g., glycosylated, phosphorylated, acylated, farnesylated, prenylated, sulfonated, and the like) to add functionality. Polypeptides exhibiting activity may be referred to as enzymes. It will be understood that, as a result of the degeneracy of the genetic code, a multitude of nucleotide sequences encoding a given polypeptide may be produced.

The term "nucleic acid sequence" (or ""polynucleotide") as used herein refers to a nucleotide polymer including at least 5 nucleotide units. A nucleic acid refers to a ribonucleotide polymer (RNA), deoxynucleotide polymer (DNA) or a modified form of either type of nucleic acid or synthetic form thereof or mixed polymers of any of the above. Nucleic acids may include either or both naturally-occurring and modified nucleic acids linked together by naturally-occurring and/or non-naturally occurring nucleic acid linkages. The nucleic acid molecules may be modified chemically or biochemically or may contain non-natural or derivatized nucleic acid bases, as will be readily appreciated by those of skill in the art. Such modifications include, for example, labels, methylation, substitution of one or more of the naturally occurring nucleic acids with an analog, internucleotide modifications such as uncharged linkages (e.g., methyl phosphonates, phosphotriesters, phosphoramidates, carbamates, etc.), charged linkages (*e.g.,* phosphorothioates, phosphorodithioates, etc.), pendent moieties (*e.g*., polypeptides), intercalators (*e.g.,* acridine, psoralen, etc.), chelators, alkylators, and modified linkages (*e.g.,* alpha anomeric nucleic acids, etc.) The term nucleic acid is also intended to include any topological conformation, including single-stranded (sense strand and antisense strand), double-stranded, partially duplexed, triplex, hairpinned, circular and padlocked conformations. Also included are synthetic molecules that mimic nucleic acids in their ability to bind to a designated sequence via hydrogen bonding and other chemical interactions. Such molecules are known in the art and include, for example, those in which peptide linkages substitute for phosphate linkages in the backbone of the molecule. A reference to a nucleic acid sequence encompasses its complement unless otherwise specified. Thus, a reference to a nucleic acid molecule having a particular sequence should be understood to encompass its complementary strand, with its complementary sequence. The complementary strand is also useful, e.g., for antisense therapy, hybridization probes and PCR primers. The term "nucleic acid", "polynucleotide" and "polynucleotide sequence" can be used interchangeably herein. A polypeptide encoded by a recombinant nucleic acid for use in a disclosed recombinant host may comprise a signal peptide and/or a propeptide sequence. In the event that a polypeptide comprises a signal peptide and/or a propeptide, sequence identity may be calculated over the mature polypeptide sequence.

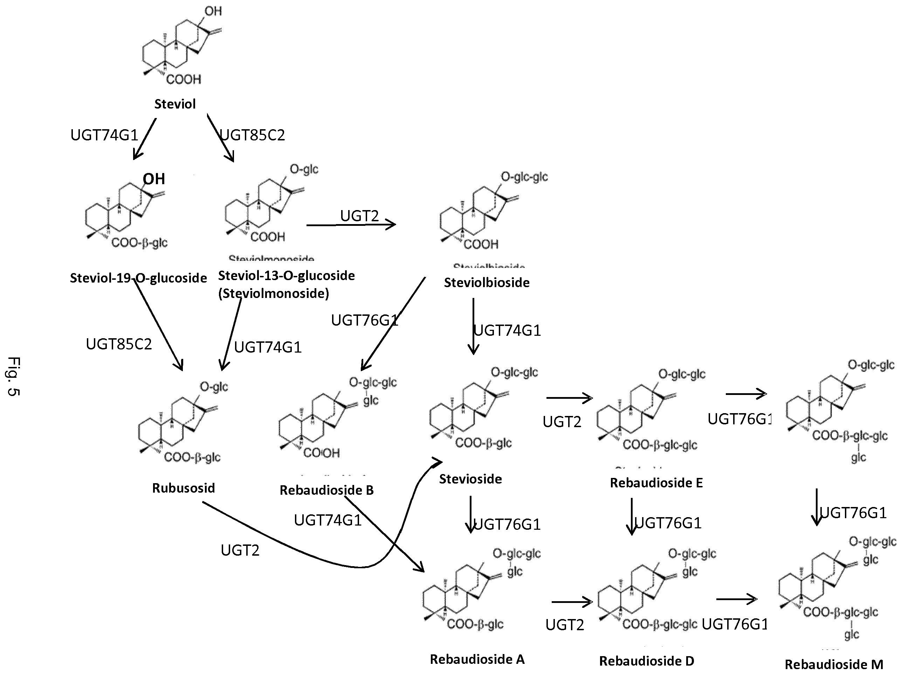

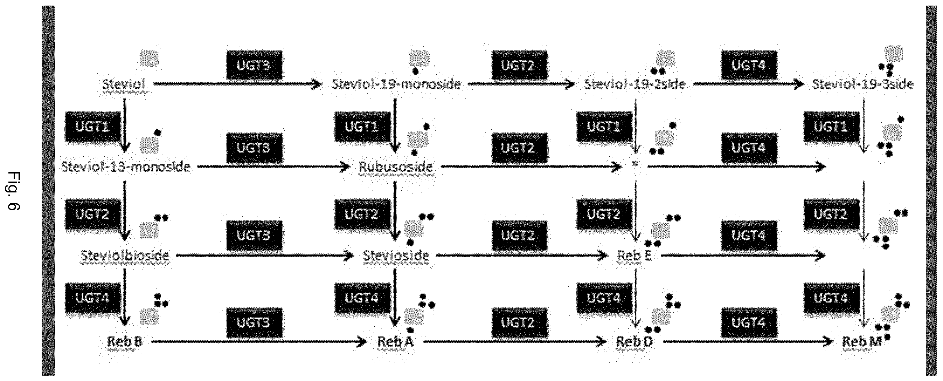

The polypeptide typically has UGT activity and more preferably has UGT2 activity. Figures 5 and 6 illustrate a non-exhaustive list of reactions that may be catalyzed by a polypeptide having UGT2 activity.

A polypeptide having UGT2 activity is one which may function as a uridine 5'-diphospho glucosyl: steviol- 13-O-glucoside transferase (also referred to as a steviol-13- monoglucoside 1,2-glucosylase), transferring a glucose moiety to the C-2' of the 13- O-glucose of the acceptor molecule, steviol- 13-O-glucoside. Typically, a suitable UGT2 polypeptide may also function as a uridine 5'-diphospho glucosyl: rubusoside transferase transferring a glucose moiety to the C-2' of the 13-0-glucose of the acceptor molecule, rubusoside. That is to say be capable of converting steviol-13-monoside to steviolbioside and/or capable of converting rubusoside to stevioside.

A polypeptide having UGT2 activity may also or alternatively catalyze reactions that utilize steviol glycoside substrates other than steviol- 13-O-glucoside and rubusoside, e.g., a functional UGT2 polypeptide may utilize stevioside as a substrate, transferring a glucose moiety to the C-2' of the 19-O-glucose residue to produce rebaudioside E. A functional UGT2 polypeptide may also or alternatively utilize rebaudioside A as a substrate, transferring a glucose moiety to the C-2' of the 19-O-glucose residue to produce rebaudioside D.

A polypeptide having UGT2 activity may also catalyze reactions that utilize steviol-19-glucoside or rubusoside as a substrate, e.g., a functional UGT2 polypeptide may utilize steviol-19-glucoside or rubusoside as a substrate, transferring a glucose moiety to the 19 position to produce steviol-19-2side or 13-[(β-D-Glucopyranosyl)oxy)kaur-16-en-18-oic acid 2-O-β-D-glucopyranosyl-β-D-glucopyranosyl ester respectively.

However, a functional UGT2 polypeptide typically does not transfer a glucose moiety to steviol compounds having a 1,3-bound glucose at the C- 13 position, i.e., transfer of a glucose moiety to steviol 1,3-bioside and 1,3-stevioside typically does not occur.

A polypeptide having UGT2 activity may also or alternatively transfer sugar moieties from donors other than uridine diphosphate glucose. For example, a polypeptide having UGT2 activity act as a uridine 5'-diphospho D-xylosyl: steviol- 13 -O-glucoside transferase, transferring a xylose moiety to the C-2' of the 13-O-glucose of the acceptor molecule, steviol- 13 -O-glucoside. As another example, a polypeptide having UGT2 activity may act as a uridine 5'-diphospho L-rhamnosyl: steviol- 13-O-glucoside transferase, transferring a rhamnose moiety to the C-2' of the 13-O-glucose of the acceptor molecule, steviol.

One or more of the above-described activities may be used to define a polypeptide having UGT2 activity encoded by a recombinant nucleic acid sequence for use in a disclosed recombinant host. Such a polypeptide may have improved UGT2 activity in respect of one or more of the above-described activities in comparison with the UGT2_1a polypeptide (SEQ ID NO: 5).

A polynucleotide encoding a polypeptide for use in a disclosed recombinant host may be used to steer production of steviol glycosides in a recombinant cell to a desired steviol glycoside, such as rebaudioside A, rebaudioside D or rebaudioside M. For example, a UGT2 polypeptide which preferentially catalyzes conversion of steviol-13-monoside to steviolbioside and/or conversion of rubusoside to stevioside may help to steer production towards rebaudiosideA, whereas a UGT2 polypeptide which preferentially catalyzes conversion of stevioside to rebE or rubusoside to a compound with an additional sugar at the 19 position may help to steer production towards rebaudioside M. That is to say preference for addition of a sugar moiety at the 13 position may help steer production towards rebaudioside A, whereas preference for addition of a sugar moiety at the 19 position may help steer production towards rebaudioside M.

A recombinant nucleic acid sequence for use in a disclosed recombinant host may be provided in the form of a nucleic acid construct. The term "nucleic acid construct" refers to as a nucleic acid molecule, either single-or double-stranded, which is isolated from a naturally occurring gene or which has been modified to contain segments of nucleic acid which are combined and juxtaposed in a manner which would not otherwise exist in nature. The term nucleic acid construct is synonymous with the term "expression cassette" when the nucleic acid construct contains all the control sequences required for expression of a coding sequence, wherein said control sequences are operably linked to said coding sequence.

A recombinant nucleic acid sequence for use in a disclosed recombinant host may be provided in the form of an expression vector, wherein the polynucleotide sequence is operably linked to at least one control sequence for the expression of the polynucleotide sequence in a recombinant host cell.

The term "operably linked" as used herein refers to two or more nucleic acid sequence elements that are physically linked and are in a functional relationship with each other. For instance, a promoter is operably linked to a coding sequence if the promoter is able to initiate or regulate the transcription or expression of a coding sequence, in which case the coding sequence should be understood as being "under the control of" the promoter. Generally, when two nucleic acid sequences are operably linked, they will be in the same orientation and usually also in the same reading frame. They usually will be essentially contiguous, although this may not be required.

An expression vector comprises a polynucleotide coding for a polypeptide as described herein, operably linked to the appropriate control sequences (such as a promoter, and transcriptional and translational stop signals) for expression and/or translation *in vitro,* or in the host cell of the polynucleotide.

The expression vector may be any vector (e.g., a plasmid or virus), which can be conveniently subjected to recombinant DNA procedures and can bring about the expression of the polynucleotide. The choice of the vector will typically depend on the compatibility of the vector with the cell into which the vector is to be introduced. The vectors may be linear or closed circular plasmids. The vector may be an autonomously replicating vector, i.e., a vector, which exists as an extra-chromosomal entity, the replication of which is independent of chromosomal replication, e.g., a plasmid, an extra-chromosomal element, a mini-chromosome, or an artificial chromosome.

Alternatively, the vector may be one which, when introduced into the host cell, is integrated into the genome and replicated together with the chromosome(s) into which it has been integrated. The integrative cloning vector may integrate at random or at a predetermined target locus in the chromosomes of the host cell. A vector may comprise one or more selectable markers, which permit easy selection of transformed cells.

Standard genetic techniques, such as overexpression of enzymes in the host cells, as well as for additional genetic modification of host cells, are known methods in the art, such as described in Sambrook and Russel (2001) "Molecular Cloning: A Laboratory Manual (3rd edition), Cold Spring Harbor Laboratory, Cold Spring Harbor Laboratory Press*,* or F. Ausubel et al, eds., "Current protocols in molecular biology", Green Publishing and Wiley Interscience, New York (1987). Methods for transformation and genetic modification of fungal host cells are known from e.g. EP-A-0 635 574, WO 98/46772, WO 99/60102 and WO 00/37671.

A disclosed recombinant host may comprise any polypeptide as described herein. Typically, a disclosed recombinant host is capable of producing a glycosylated diterpene, such as a steviol glycoside. For example, a disclosed recombinant host may be capable of producing one or more of, for example, steviol-13-monoside, steviol-19-monoside, 13-[(β-D-Glucopyranosyl)oxy)kaur-16-en-18-oic acid 2-O-β-D-glucopyranosyl-β-D-glucopyranosyl ester, rubusoside, stevioside, steviol-19-diside, steviolbioside, rebA, rebE, rebD or rebM.

Thus, a disclosed recombinant host will typically comprise polynucleotides encoding polypeptides having UGT1, UGT2, UGT2 and UGT4 activity and polypeptides which provide for the production of steviol in the host (which may then be converted to one or more steviol glycosides).

One polynucleotide may encode more than one of such polypeptides. One polynucleotide may encode a polypeptide having more than one of the activities UGT1, UGT2, UGT3 or UGT4 or the activity of a polypeptide providing for production of steviol in the host. Accordingly, a disclosed recombinant host may comprise one or more recombinant nucleotide sequence(s) encoding one of more of:
a polypeptide having ent-copalyl pyrophosphate synthase activity;
a polypeptide having ent-Kaurene synthase activity;
a polypeptide having ent-Kaurene oxidase activity; and
a polypeptide having kaurenoic acid 13-hydroxylase activity.

A recombinant host may comprise one or more recombinant polynucleotide sequences encoding all four such polypeptides.

Herein, a polypeptide having *ent*-copalyl pyrophosphate synthase (EC 5.5.1.13) is capable of catalyzing the chemical reation:

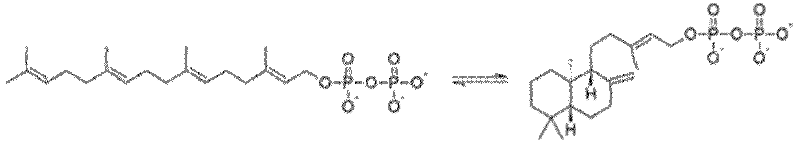

This enzyme has one substrate, geranylgeranyl pyrophosphate, and one product, *ent-*copalyl pyrophosphate. This enzyme participates in gibberellin biosynthesis. This enzyme belongs to the family of isomerase, specifically the class of intramolecular lyases. The systematic name of this enzyme class is *ent*-copalyl-diphosphate lyase (decyclizing). Other names in common use include having *ent*-copalyl pyrophosphate synthase, *ent*-kaurene synthase A, and *ent*-kaurene synthetase A.

Suitable nucleic acid sequences encoding an ent-copalyl pyrophosphate synthase may for instance comprise a sequence as set out in SEQ ID. NO: 1, 3, 5, 7, 17, 19, 59, 61, 141, 142, 151, 152, 153, 154, 159, 160, 182 or 184 of WO2015/007748.

Herein, a polypeptide having ent-kaurene synthase activity (EC 4.2.3.19) is a polypeptide that is capable of catalyzing the chemical reaction:

ent-copalyl diphosphate ⇄ent-kaurene + diphosphate

Hence, this enzyme has one substrate, ent-copalyl diphosphate, and two products, ent-kaurene and diphosphate.

This enzyme belongs to the family of lyases, specifically those carbon-oxygen lyases acting on phosphates. The systematic name of this enzyme class is ent-copalyl-diphosphate diphosphate-lyase (cyclizing, ent-kaurene-forming). Other names in common use include ent-kaurene synthase B, ent-kaurene synthetase B, ent-copalyl-diphosphate diphosphate-lyase, and (cyclizing). This enzyme participates in diterpenoid biosynthesis.

Suitable nucleic acid sequences encoding an ent-Kaurene synthase may for instance comprise a sequence as set out in SEQ ID. NO: 9, 11, 13, 15, 17, 19, 63, 65, 143, 144, 155, 156, 157, 158, 159, 160, 183 or 184 of WO2015/007748.

*ent*-copalyl diphosphate synthases may also have a distinct *ent*-kaurene synthase activity associated with the same protein molecule. The reaction catalyzed by *ent*-kaurene synthase is the next step in the biosynthetic pathway to gibberellins. The two types of enzymic activity are distinct, and site-directed mutagenesis to suppress the *ent*-kaurene synthase activity of the protein leads to build up of *ent*-copalyl pyrophosphate.

Accordingly, a single nucleotide sequence used in a disclosed recombinant host may encode a polypeptide having *ent*-copalyl pyrophosphate synthase activity and *ent*-kaurene synthase activity. Alternatively, the two activities may be encoded two distinct, separate nucleotide sequences.

Herein, a polypeptide having ent-kaurene oxidase activity (EC 1.14.13.78) is a polypeptide which is capable of catalysing three successive oxidations of the 4-methyl group of ent-kaurene to give kaurenoic acid. Such activity typically requires the presence of a cytochrome P450.

Suitable nucleic acid sequences encoding an ent-Kaurene oxidase may for instance comprise a sequence as set out in SEQ ID. NO: 21, 23, 25, 67, 85, 145, 161, 162, 163, 180 or 186 of WO2015/007748.

Herein, a polypeptide having kaurenoic acid 13-hydroxylase activity (EC 1.14.13) is one which is capable of catalyzing the formation of steviol (ent-kaur-16-en-13-ol-19-oic acid) using NADPH and O₂. Such activity may also be referred to as ent-ka 13-hydroxylase activity.

Suitable nucleic acid sequences encoding a kaurenoic acid 13-hydroxylase may for instance comprise a sequence as set out in SEQ ID. NO: 27, 29, 31, 33, 69, 89, 91, 93, 95, 97, 146, 164, 165, 166, 167 or 185 of WO2015/007748.

A disclosed recombinant host may comprise a recombinant nucleic acid sequence encoding a polypeptide having NADPH-cytochrome p450 reductase activity. That is to say, a recombinant host of the invention may be capable of expressing a nucleotide sequence encoding a polypeptide having NADPH-cytochrome p450 reductase activity. For the purposes of the invention, a polypeptide having NADPH-Cytochrome P450 reductase activity (EC 1.6.2.4; also known as NADPH:ferrihemoprotein oxidoreductase, NADPH:hemoprotein oxidoreductase, NADPH:P450 oxidoreductase, P450 reductase, POR, CPR, CYPOR) is typically one which is a membrane-bound enzyme allowing electron transfer to cytochrome P450 in the microsome of the eukaryotic cell from a FAD- and FMN-containing enzyme NADPH:cytochrome P450 reductase (POR; EC 1.6.2.4).

A disclosed recombinant host may comprise one or more recombinant nucleic acid sequences encoding one or more UGT polypeptides, in addition to RT7, RT11, RT15 or RT18 or related sequences as described herein. Such additional UGTs may be selected so as to produce a desired diterpene glycoside, such as a steviol glycoside. Schematic diagrams of steviol glycoside formation are set out in Humphrey et al., Plant Molecular Biology (2006) 61: 47-62 and Mohamed et al., J. Plant Physiology 168 (2011) 1136-1141. In addition, Figures 5 and 6 sets out a schematic diagram of steviol glycoside formation.

A disclosed recombinant host may thus comprise one or more recombinant nucleic acid sequences encoding one or more of:
(i) a polypeptide having UGT74G1 activity (UGT3 activity);
(ii) a polypeptide having UGT85C2 activity (UGT1 activity); and
(iii) a polypeptide having UGT76G1 activity (UGT4 activity).

Figures 5 and 6 set out schematic diagram of the potential pathways leading to biosynthesis of steviol glycosides.

A disclosed recombinant host will typically comprise at least one recombinant nucleic acid encoding a polypeptide having UGT1 activity, at least one recombinant nucleic acid encoding a polypeptide having UGT2 activity, at least one recombinant nucleic acid encoding a polypeptide having UGT3 activity and at least one recombinant nucleic acid encoding a polypeptide having UGT4 activity. One nucleic acid may encode two or more of such polypeptides.

A disclosed recombinant host typically comprises polynucleotides expressing at least one of each of a UGT1, UGT2, UGT3 and UGT4 polypeptide and a polypeptide having ent-copalyl pyrophosphate synthase activity, a polypeptide having ent-Kaurene synthase activity, a polypeptide having ent-Kaurene oxidase activity and a polypeptide having kaurenoic acid 13-hydroxylase activity. In such a recombinant host, all polynucleotides encoding such polypeptides may be recombinant.

A nucleic acid encoding a polypeptide as described herein may be used to steer production of steviol glycosides in a recombinant cell to a desired steviol glycoside, such as rebaudioside A, rebaudioside D or rebaudioside M. For example, a recombinant nucleic acid which encodes a UGT2 polypeptide which preferentially catalyzes conversion of steviol-13-monoside to steviolbioside and/or conversion of rubusoside to stevioside may help to steer production towards rebaudiosideA, whereas a recombinant nucleic acid which encodes a UGT2 polypeptide which preferentially catalyzes conversion of stevioside to rebE or rubusoside to a compound with an additional sugar at the 19 position may help to steer production towards rebaudioside M. That is to say preference for addition of a sugar moiety at the 13 position may help steer production towards rebaudioside A, whereas preference for addition of a sugar moiety at the 19 position may help steer production towards rebaudioside M. A disclosed recombinant host may comprises a nucleotide sequence encoding a polypeptide capable of catalyzing the addition of a C-13-glucose to steviol. That is to say, a disclosed recombinant host may comprise a UGT which is capable of catalyzing a reaction in which steviol is converted to steviolmonoside.

Such a disclosed recombinant host may comprise a nucleotide sequence encoding a polypeptide having the activity shown by UDP-glycosyltransferase (UGT) UGT85C2, whereby the nucleotide sequence upon transformation of the host confers on that host the ability to convert steviol to steviolmonoside.

UGT85C2 activity is transfer of a glucose unit to the 13-OH of steviol. Thus, a suitable UGT85C2 may function as a uridine 5'-diphospho glucosyl: steviol 13-OH transferase, and a uridine 5'-diphospho glucosyl: steviol- 19-O-glucoside 13-OH transferase. A functional UGT85C2 polypeptides may also catalyze glucosyl transferase reactions that utilize steviol glycoside substrates other than steviol and steviol- 19-O-glucoside. Such sequences may be referred to as UGT1 sequences herein.

A disclosed recombinant host may comprises a nucleotide sequence encoding a polypeptide having UGT activity may comprise a nucleotide sequence encoding a polypeptide capable of catalyzing the addition of a C-19-glucose to steviolbioside and/or to rebaudioside B. That is to say, a disclosed recombinant host may comprise a UGT which is capable of catalyzing a reaction in which steviolbioside is converted to stevioside and/or in which rebaudioside B is converted to rebaudioside A. Accordingly, such a recombinant host may be capable of converting steviolbioside to stevioside and/or rebaudioside B is converted to rebaudioside A. Expression of such a nucleotide sequence may confer on the recombinant host the ability to produce at least stevioside and/or rebaudioside A.

A disclosed recombinant host may thus also comprise a nucleotide sequence encoding a polypeptide having the activity shown by UDP-glycosyltransferase (UGT) UGT74G1, whereby the nucleotide sequence upon transformation of the host confers on the cell the ability to convert steviolbioside to stevioside.

Suitable UGT74G1 polypeptides may be capable of transferring a glucose unit to the 13-OH or the 19-COOH, respectively, of steviol. A suitable UGT74G1 polypeptide may function as a uridine 5'-diphospho glucosyl: steviol 19-COOH transferase and a uridine 5'-diphospho glucosyl: steviol- 13-O-glucoside 19-COOH transferase. Functional UGT74G1 polypeptides also may catalyze glycosyl transferase reactions that utilize steviol glycoside substrates other than steviol and steviol- 13-0-glucoside, or that transfer sugar moieties from donors other than uridine diphosphate glucose. Such sequences may be referred to herein as UGT3 sequences.

A disclosed recombinant host may comprise a nucleotide sequence encoding a polypeptide capable of catalyzing glucosylation of the C-3' of the glucose at the C-13 position of stevioside. That is to say, a disclosed recombinant host may comprise a UGT which is capable of catalyzing a reaction in which stevioside is converted to rebaudioside A. Accordingly, such a recombinant host may be capable of converting stevioside to rebaudioside A. Expression of such a nucleotide sequence may confer on the host the ability to produce at least rebaudioside A.

A disclosed recombinant host may thus also comprise a nucleotide sequence encoding a polypeptide having the activity shown by UDP-glycosyltransferase (UGT) UGT76G1, whereby the nucleotide sequence upon transformation of a host confers on that host the ability to convert stevioside to rebaudioside A and/or steviolbioside to rebaudioside B.

A suitable UGT76G1 adds a glucose moiety to the C-3' of the C-13-O-glucose of the acceptor molecule, a steviol 1,2 glycoside. Thus, UGT76G1 functions, for example, as a uridine 5'-diphospho glucosyl: steviol 13-0-1,2 glucoside C-3 ' glucosyl transferase and a uridine 5'-diphospho glucosyl: steviol- 19-O-glucose, 13-0-1,2 bioside C-3' glucosyl transferase. Functional UGT76G1 polypeptides may also catalyze glucosyl transferase reactions that utilize steviol glycoside substrates that contain sugars other than glucose, e.g., steviol rhamnosides and steviol xylosides. Such sequences may be referred to herein as UGT4 sequences. A UGT4 may alternatively or in addition be capable of converting RebD to RebM.

A disclosed recombinant host typically comprises nucleotide sequences encoding polypeptides having all four UGT activities described above. A given nucleic acid may encode a polypeptide having one or more of the above activities. For example, a nucleic acid encode for a polypeptide which has two, three or four of the activities set out above. Preferably, a disclosed recombinant host comprises UGT1, UGT2 and UGT3 and UGT4 activity. Suitable UGT1, UGT3 and UGT4 sequences are described in in Table 1 of WO2015/007748.

A disclosed recombinant host may comprise a recombinant nucleic acid sequence encoding an additional polypeptide having UGT2 activity. That is to say, a disclosed recombinant host may comprise a nucleic acid sequence encoding a variant UGT2 and one or more additional, different, variant or any another, different, UGT2.

Use of a nucleic acid sequence encoding a RT7, RT11, RT15 or RT18 polypeptide (or related polypeptide as described herein) may be useful in improving rebA production in a disclosed recombinant host.

Use of a nucleic acid sequence encoding a RT7, RT11, RT15 or RT18 polypeptide (or related polypeptide as described herein) may be useful in improving rebM production in a disclosed recombinant host.

In a disclosed recombinant host, the ability of the host to produce geranylgeranyl diphosphate (GGPP) may be upregulated. Upregulated in the context of this invention implies that the recombinant host produces more GGPP than an equivalent non-recombinant host.

Accordingly, a disclosed recombinant host may comprise one or more nucleotide sequence(s) encoding hydroxymethylglutaryl-CoA reductase, farnesyl-pyrophosphate synthetase and geranylgeranyl diphosphate synthase, whereby the nucleotide sequence(s) upon transformation of a host confer(s) on that host the ability to produce elevated levels of GGPP. Thus, a disclosed recombinant host may comprise one or more recombinant nucleic acid sequence(s) encoding one or more of hydroxymethylglutaryl-CoA reductase, farnesyl-pyrophosphate synthetase and geranylgeranyl diphosphate synthase.

Accordingly, a disclosed recombinant host may comprise nucleic acid sequences encoding one or more of:
a polypeptide having hydroxymethylglutaryl-CoA reductase activity;
a polypeptide having farnesyl-pyrophosphate synthetase activity;
a polypeptide having geranylgeranyl diphosphate synthase activity.

A disclosed recombinant host may be, for example, an multicellular organism or a cell thereof or a unicellular organism. A disclosed host may be a prokaryotic, archaebacterial or eukaryotic host cell.

A prokaryotic host cell may, but is not limited to, a bacterial host cell. An eukaryotic host cell may be, but is not limited to, a yeast, a fungus, an amoeba, an algae, an animal, an insect or a plant host cell.

An eukaryotic host cell may be a fungal host cell. "Fungi" include all species of the subdivision *Eumycotina* (Alexopoulos, C. J., 1962, In: Introductory Mycology, John Wiley & Sons, Inc., New York). The term fungus thus includes among others filamentous fungi and yeast.

"Filamentous fungi" are herein defined as eukaryotic microorganisms that include all filamentous forms of the subdivision *Eumycotina* and *Oomycota* (as defined by Hawksworth *et al.,* 1995, supra). The filamentous fungi are characterized by a mycelial wall composed of chitin, cellulose, glucan, chitosan, mannan, and other complex polysaccharides. Vegetative growth is by hyphal elongation and carbon catabolism is obligatory aerobic. Filamentous fungal strains include, but are not limited to, strains of *Acremonium, Aspergillus, Agaricus, Aureobasidium, Cryptococcus, Corynascus, Chrysosporium, Filibasidium, Fusarium, Humicola, Magnaporthe, Monascus, Mucor, Myceliophthora, Mortierella, Neocallimastix, Neurospora, Paecilomyces, Penicillium, Piromyces, Phanerochaete Podospora, Pycnoporus, Rhizopus, Schizophyllum, Sordaria, Talaromyces, Rasmsonia, Thermoascus, Thielavia, Tolypocladium, Trametes* and *Trichoderma.* Preferred filamentous fungal strains that may serve as host cells belong to the species *Aspergillus niger, Aspergillus oryzae, Aspergillus fumigatus, Penicillium chrysogenum, Penicillium citrinum, Acremonium chrysogenum, Trichoderma reesei, Rasamsonia emersonii* (formerly known as *Talaromyces emersonii*), *Aspergillus sojae, Chrysosporium lucknowense, Myceliophtora thermophyla.* Reference host cells for the comparison of fermentation characteristics of transformed and untransformed cells, include e.g. *Aspergillus niger* CBS120.49, CBS 513.88, *Aspergillus oryzae* ATCC16868, ATCC 20423, IFO 4177, ATCC 1011, ATCC 9576, ATCC14488-14491, ATCC 11601, ATCC12892, *Aspergillus fumigatus* AF293 (CBS101355), *P. chrysogenum* CBS 455.95, *Penicillium citrinum* ATCC 38065, *Penicillium chrysogenum* P2, *Acremonium chrysogenum* ATCC 36225, ATCC 48272, *Trichoderma reesei* ATCC 26921, ATCC 56765, ATCC 26921, *Aspergillus sojae* ATCC11906, *Chrysosporium lucknowense* ATCC44006 and derivatives of all of these strains. Particularly preferred as filamentous fungal host cell are *Aspergillus niger* CBS 513.88 and derivatives thereof.

An eukaryotic host cell may be a yeast cell. Preferred yeast host cells may be selected from the genera: *Saccharomyces* (e.g., S. *cerevisiae, S. bayanus, S. pastorianus, S. carlsbergensis), Brettanomyces, Kluyveromyces, Candida* (e.g., C. *krusei, C. revkaufi, C. pulcherrima, C. tropicalis, C. utilis), Issatchenkia* (eg. *I. orientalis) Pichia* (e.g., *P. pastoris*)*, Schizosaccharomyces, Hansenula, Kloeckera, Pachysolen, Schwanniomyces, Trichosporon, Yarrowia* (e.g., *Y. lipolytica* (formerly classified as *Candida lipolytica*)), *Yamadazyma* .

Prokaryotic host cells may be bacterial host cells. Bacterial host cell may be Gram negative or Gram positive bacteria. Examples of bacteria include, but are not limited to, bacteria belonging to the genus *Bacillus* (e.g., *B. subtilis, B. amyloliquefaciens, B. licheniformis, B. puntis, B. megaterium, B. halodurans, B. pumilus,*)*, Acinetobacter, Nocardia, Xanthobacter, Escherichia* (e.g., *E. coli* (e.g., strains DH 1 OB, Stbl2, DH5-alpha, DB3, DB3.1), DB4, DB5, JDP682 and ccdA-over (e.g., U.S. application No. 09/518,188))), *Streptomyces, Erwinia, Klebsiella, Serratia* (e.g., *S. marcessans), Pseudomonas* (e.g., *P. aeruginosa), Salmonella* (e.g., *S. typhimurium, S. typhi*)*.* Bacteria also include, but are not limited to, photosynthetic bacteria (e.g., green non-sulfur bacteria (e.g., *Choroflexus* bacteria (e.g., *C. aurantiacus*)*, Chloronema* (e.g., *C. gigateum*)), green sulfur bacteria (e.g., *Chlorobium bacteria* (e.g., *C. limicola*), *Pelodictyon* (e.g., *P. luteolum),* purple sulfur bacteria (e.g., *Chromatium* (e.g., *C. okenii*)), and purple non-sulfur bacteria (e.g., *Rhodospirillum* (e.g., *R. rubrum), Rhodobacter* (e.g. *R. sphaeroides, R. capsulatus*)*,* and *Rhodomicrobium* bacteria (e.g., *R. vanellii*)).

Host cells may be host cells from non-microbial organisms. Examples of such cells, include, but are not limited to, insect cells (e.g., *Drosophila* (e.g., *D. melanogaster*)*, Spodoptera* (e.g., *S. frugiperda* Sf9 or Sf21 cells) and *Trichoplusa* (e.g., High-Five cells); nematode cells (e.g., *C. elegans* cells); avian cells; amphibian cells (e.g., *Xenopus laevis* cells); reptilian cells; and mammalian cells (e.g., NIH3T3, 293, CHO, COS, VERO, C127, BHK, Per-C6, Bowes melanoma and HeLa cells).

A disclosed recombinant host may be able to grow on any suitable carbon source known in the art and convert it to a glycosylated diterpene, eg. a steviol glycoside. The recombinant host may be able to convert directly plant biomass, celluloses, hemicelluloses, pectines, rhamnose, galactose, fucose, maltose, maltodextrines, ribose, ribulose, or starch, starch derivatives, sucrose, lactose and glycerol. Hence, a preferred host expresses enzymes such as cellulases (endocellulases and exocellulases) and hemicellulases (e.g. endo- and exo-xylanases, arabinases) necessary for the conversion of cellulose into glucose monomers and hemicellulose into xylose and arabinose monomers, pectinases able to convert pectines into glucuronic acid and galacturonic acid or amylases to convert starch into glucose monomers. Preferably, the host is able to convert a carbon source selected from the group consisting of glucose, xylose, arabinose, sucrose, lactose and glycerol. The host cell may for instance be a eukaryotic host cell as described in WO03/062430, WO06/009434, EP1499708B1, WO2006096130 or WO04/099381.

Thus, further disclosed is a process for the preparation of a glycosylated diterpene which comprises fermenting a disclosed recombinant host which is capable of producing at least one glycosylated diterpene in a suitable fermentation medium, and optionally recovering the glycosylated diterpene.

The fermentation medium used in the process for the production of a glycosylated diterpene may be any suitable fermentation medium which allows growth of a particular eukaryotic host cell. The essential elements of the fermentation medium are known to the person skilled in the art and may be adapted to the host cell selected.

Preferably, the fermentation medium comprises a carbon source selected from the group consisting of plant biomass, celluloses, hemicelluloses, pectines, rhamnose, galactose, fucose, fructose, maltose, maltodextrines, ribose, ribulose, or starch, starch derivatives, sucrose, lactose, fatty acids, triglycerides and glycerol. Preferably, the fermentation medium also comprises a nitrogen source such as ureum, or an ammonium salt such as ammonium sulphate, ammonium chloride, ammoniumnitrate or ammonium phosphate.

The disclosed fermentation process may be carried out in batch, fed-batch or continuous mode. A separate hydrolysis and fermentation (SHF) process or a simultaneous saccharification and fermentation (SSF) process may also be applied. A combination of these fermentation process modes may also be possible for optimal productivity. A SSF process may be particularly attractive if starch, cellulose, hemicelluose or pectin is used as a carbon source in the fermentation process, where it may be necessary to add hydrolytic enzymes, such as cellulases, hemicellulases or pectinases to hydrolyse the substrate.

The recombinant host used in the process for the preparation of a glycosylated diterpene may be any suitable recombinant host as defined herein above. It may be advantageous to use a disclosed recombinant eukaryotic recombinant host in the process since most eukaryotic cells do not require sterile conditions for propagation and are insensitive to bacteriophage infections. In addition, eukaryotic host cells may be grown at low pH to prevent bacterial contamination.

The disclosed recombinant host may be a facultative anaerobic microorganism. A facultative anaerobic recombinant host can be propagated aerobically to a high cell concentration. This anaerobic phase can then be carried out at high cell density which reduces the fermentation volume required substantially, and may minimize the risk of contamination with aerobic microorganisms.

The fermentation process for the production of a disclosed glycosylated diterpene may be an aerobic or an anaerobic fermentation process.

An anaerobic fermentation process may be herein defined as a fermentation process run in the absence of oxygen or in which substantially no oxygen is consumed, preferably less than 5, 2.5 or 1 mmol/L/h, and wherein organic molecules serve as both electron donor and electron acceptors. The disclosed fermentation process according to the present invention may also first be run under aerobic conditions and subsequently under anaerobic conditions.

The fermentation process may also be run under oxygen-limited, or micro-aerobical, conditions. Alternatively, the fermentation process may first be run under aerobic conditions and subsequently under oxygen-limited conditions. An oxygen-limited fermentation process is a process in which the oxygen consumption is limited by the oxygen transfer from the gas to the liquid. The degree of oxygen limitation is determined by the amount and composition of the ingoing gasflow as well as the actual mixing/mass transfer properties of the fermentation equipment used.

The production of a glycosylated diterpene in the disclosed process may occur during the growth phase of the host cell, during the stationary (steady state) phase or during both phases. It may be possible to run the fermentation process at different temperatures.

The process for the production of a glycosylated diterpene may be run at a temperature which is optimal for the recombinant host. The optimum growth temperature may differ for each transformed recombinant host and is known to the person skilled in the art. The optimum temperature might be higher than optimal for wild type organisms to grow the organism efficiently under non-sterile conditions under minimal infection sensitivity and lowest cooling cost. Alternatively, the process may be carried out at a temperature which is not optimal for growth of the recombinant host.

The process for the production of a glycosylated diterpene disclosed may be carried out at any suitable pH value. If the recombinant host is a yeast, the pH in the fermentation medium preferably has a value of below 6, preferably below 5,5, preferably below 5, preferably below 4,5, preferably below 4, preferably below pH 3,5 or below pH 3,0, or below pH 2,5, preferably above pH 2. An advantage of carrying out the fermentation at these low pH values is that growth of contaminant bacteria in the fermentation medium may be prevented.

Such a process may be carried out on an industrial scale. The product of such a process is one or more glycosylated diterpenes, such as one or more steviol glycosides.

Recovery of glycosylated diterpene(s) from the fermentation medium may be performed by known methods in the art, for instance by distillation, vacuum extraction, solvent extraction, or evaporation.

In the process for the production of a disclosed glycosylated diterpene, it may be possible to achieve a concentration of above 5 mg/l fermentation broth, preferably above 10 mg/l, preferably above 20 mg/l, preferably above 30 mg/l fermentation broth, preferably above 40 mg/l, more preferably above 50 mg/l, preferably above 60 mg/l, preferably above 70, preferably above 80 mg/l, preferably above 100 mg/l, preferably above 1 g/l, preferably above 5 g/l, preferably above 10 g/l, for example above 20g/l, but usually up to a concentration of about 200g/l, such as up to about 150g/l, such as up to about 100g/l, for example up to about 70 g/l. Such concentrations may be concentration of the total broth or of the supernatant..

Further disclosed is a fermentation broth comprising a glycosylated diterpene obtainable by the process of the invention for the preparation of a glycosylated diterpene.

In the event that one or more glycosylated diterpenes is expressed within a disclosed recombinant, such cells may need to be treated so as to release them. Preferentially, at least one glycosylated diterpene, such as a steviol glycoside, for example rebA or rebM, is produced extracellularly

Further disclosed is a glycosylated diterpene obtained by a disclosed process for the preparation of a glycosylated diterpene or obtainable from a disclosed fermentation broth. Such a glycosylated diterpene may be a non- naturally occurring glycosylated diterpene, that is to say one which is not produced in plants.

Also disclosed is a composition comprising one or more steviol glycosides obtainable by process for the preparation of a glycosylated diterpene or obtainable from a disclosed fermentation broth. Such a composition may comprise two or more glycosylated diterpenes obtainable by a disclosed process for the preparation of a glycosylated diterpene or obtainable from a disclosed fermentation broth. In such a composition, one or more of the glycosylated diterpenes may be a non-naturally occurring glycosylated diterpene, that is to say one which is not produced in plants.

Further disclosed is a method for converting a first glycosylated diterpene into a second glycosylated diterpene, which method comprises:
- contacting said first glycosylated diterpene with a disclosed recombinant host, a cell free extract derived from such a recombinant host or an enzyme preparation derived from either thereof;
- thereby to convert the first glycosylated diterpene into the second glycosylated diterpene.

In such a method, the second glycosylated diterpene may be steviol-19-diside, steviolbioside, stevioside, 13-[(β-D-Glucopyranosyl)oxy)kaur-16-en-18-oic acid 2-O-β-D-glucopyranosyl-p-D-glucopyranosyl ester, RebE or RebD.

In such a method, the first glycosylated diterpene may be steviol-13-monoside, steviol-19-monoside, rubusoside, stevioside, rebaudioside A or 13-[(β-D-Glucopyranosyl)oxy)kaur-16-en-18-oic acid 2-O-β-D-glucopyranosyl-β-D-glucopyranosyl ester and the second glycosylated diterpene is steviol-19-diside, steviolbioside, stevioside, 13-[(β-D-Glucopyranosyl)oxy)kaur-16-en-18-oic acid 2-O-β-D-glucopyranosyl-β-D-glucopyranosyl ester, RebE or RebD.

These are the first and second steviol glycosides in relation to a reaction catalysed by a polypeptide described herein having UGT2 activity.

That is to say, the disclosure also relates to a method of bioconversion or biotransformation.

A steviol glycoside or composition produced by a disclosed fermentation process may be used in any application known for such compounds. In particular, they may for instance be used as a sweetener, for example in a food or a beverage. Also disclosed is a foodstuff, feed or beverage which comprises a glycosylated diterpene, such as a steviol glycoside, or a composition of the invention.

For example a disclosed glycosylated diterpene or a disclosed composition may be formulated in soft drinks, as a tabletop sweetener, chewing gum, dairy product such as yoghurt (eg. plain yoghurt), cake, cereal or cereal-based food, nutraceutical, pharmaceutical, edible gel, confectionery product, cosmetic, toothpastes or other oral cavity composition, etc. In addition, a disclosed glycosylated diterpene or a disclosed composition can be used as a sweetener not only for drinks, foodstuffs, and other products dedicated for human consumption, but also in animal feed and fodder with improved characteristics.

Accordingly, disclosed is, *inter alia,* a foodstuff, feed or beverage which comprises a diterpene or glycosylated diterpene prepared according to a process of the invention.

During the manufacturing of foodstuffs, drinks, pharmaceuticals, cosmetics, table top products, chewing gum the conventional methods such as mixing, kneading, dissolution, pickling, permeation, percolation, sprinkling, atomizing, infusing and other methods can be used.

The glycosylated diterpene, for example a steviol glycoside, or a disclosed composition can be used in dry or liquid forms. It can be added before or after heat treatment of food products. The amount of the sweetener depends on the purpose of usage. It can be added alone or in the combination with other compounds.

Compounds produced according to the disclosed method may be blended with one or more further non-caloric or caloric sweeteners. Such blending may be used to improve flavour or temporal profile or stability. A wide range of both non-caloric and caloric sweeteners may be suitable for blending with a disclosed glycosylated diterpene or a disclosed composition. For example, non-caloric sweeteners such as mogroside, monatin, aspartame, acesulfame salts, cyclamate, sucralose, saccharin salts or erythritol. Caloric sweeteners suitable for blending with a glycosylated diterpene or a composition of the invention include sugar alcohols and carbohydrates such as sucrose, glucose, fructose and HFCS. Sweet tasting amino acids such as glycine, alanine or serine may also be used.

A disclosed glycosylated diterpene or a disclosed composition can be used in the combination with a sweetener suppressor, such as a natural sweetener suppressor. It may be combined with an umami taste enhancer, such as an amino acid or a salt thereof.

A disclosed glycosylated diterpene or a disclosed composition can be combined with a polyol or sugar alcohol, a carbohydrate, a physiologically active substance or functional ingredient (for example a carotenoid, dietary fiber, fatty acid, saponin, antioxidant, nutraceutical, flavonoid, isothiocyanate, phenol, plant sterol or stanol (phytosterols and phytostanols), a polyols, a prebiotic, a probiotic, a phytoestrogen, soy protein, sulfides/thiols, amino acids, a protein, a vitamin, a mineral, and/or a substance classified based on a health benefits, such as cardiovascular, cholesterol-reducing or anti-inflammatory.

A composition with a glycosylated diterpene or a disclosed composition may include a flavoring agent, an aroma component, a nucleotide, an organic acid, an organic acid salt, an inorganic acid, a bitter compound, a protein or protein hydrolyzate, a surfactant, a flavonoid, an astringent compound, a vitamin, a dietary fiber, an antioxidant, a fatty acid and/or a salt.

A disclosed glycosylated diterpene or a disclosed composition may be applied as a high intensity sweetener to produce zero calorie, reduced calorie or diabetic beverages and food products with improved taste characteristics. Also it can be used in drinks, foodstuffs, pharmaceuticals, and other products in which sugar cannot be used.

In addition, a disclosed glycosylated diterpene or a disclosed composition may be used as a sweetener not only for drinks, foodstuffs, and other products dedicated for human consumption, but also in animal feed and fodder with improved characteristics.

The examples of products where a disclosed glycosylated diterpene or a disclosed composition can be used as a sweetening compound can be as alcoholic beverages such as vodka, wine, beer, liquor, sake, etc; natural juices, refreshing drinks, carbonated soft drinks, diet drinks, zero calorie drinks, reduced calorie drinks and foods, yogurt drinks, instant juices, instant coffee, powdered types of instant beverages, canned products, syrups, fermented soybean paste, soy sauce, vinegar, dressings, mayonnaise, ketchups, curry, soup, instant bouillon, powdered soy sauce, powdered vinegar, types of biscuits, rice biscuit, crackers, bread, chocolates, caramel, candy, chewing gum, jelly, pudding, preserved fruits and vegetables, fresh cream, jam, marmalade, flower paste, powdered milk, ice cream, sorbet, vegetables and fruits packed in bottles, canned and boiled beans, meat and foods boiled in sweetened sauce, agricultural vegetable food products, seafood, ham, sausage, fish ham, fish sausage, fish paste, deep fried fish products, dried seafood products, frozen food products, preserved seaweed, preserved meat, tobacco, medicinal products, and many others. In principal it can have unlimited applications.

The sweetened composition comprises a beverage, non-limiting examples of which include non-carbonated and carbonated beverages such as colas, ginger ales, root beers, ciders, fruit-flavored soft drinks (e.g., citrus-flavored soft drinks such as lemon-lime or orange), powdered soft drinks, and the like; fruit juices originating in fruits or vegetables, fruit juices including squeezed juices or the like, fruit juices containing fruit particles, fruit beverages, fruit juice beverages, beverages containing fruit juices, beverages with fruit flavorings, vegetable juices, juices containing vegetables, and mixed juices containing fruits and vegetables; sport drinks, energy drinks, near water and the like drinks (e.g., water with natural or synthetic flavorants); tea type or favorite type beverages such as coffee, cocoa, black tea, green tea, oolong tea and the like; beverages containing milk components such as milk beverages, coffee containing milk components, cafe au lait, milk tea, fruit milk beverages, drinkable yogurt, lactic acid bacteria beverages or the like; and dairy products.

Generally, the amount of sweetener present in a sweetened composition varies widely depending on the particular type of sweetened composition and its desired sweetness. Those of ordinary skill in the art can readily discern the appropriate amount of sweetener to put in the sweetened composition.

A disclosed glycosylated diterpene or a disclosed composition can be used in dry or liquid forms. It can be added before or after heat treatment of food products. The amount of the sweetener depends on the purpose of usage. It can be added alone or in the combination with other compounds.

During the manufacturing of foodstuffs, drinks, pharmaceuticals, cosmetics, table top products, chewing gum the conventional methods such as mixing, kneading, dissolution, pickling, permeation, percolation, sprinkling, atomizing, infusing and other methods can be used.

Thus, disclosed compositions can be made by any method known to those skilled in the art that provide homogenous even or homogeneous mixtures of the ingredients. These methods include dry blending, spray drying, agglomeration, wet granulation, compaction, co-crystallization and the like.

In solid form a disclosed glycosylated diterpene or a disclosed composition can be provided to consumers in any form suitable for delivery into the comestible to be sweetened, including sachets, packets, bulk bags or boxes, cubes, tablets, mists, or dissolvable strips. The composition can be delivered as a unit dose or in bulk form.

For liquid sweetener systems and compositions convenient ranges of fluid, semi-fluid, paste and cream forms, appropriate packing using appropriate packing material in any shape or form shall be invented which is convenient to carry or dispense or store or transport any combination containing any of the above sweetener products or combination of product produced above.

The composition may include various bulking agents, functional ingredients, colorants, flavors.

The terms "sequence homology" or "sequence identity" or "homology" or "identity" are used interchangeably herein. Herein, it is defined here that in order to determine the percentage of sequence homology or sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. The sequence identity is the percentage of identical matches between the two sequences over the reported aligned region.

A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley). The percent sequence identity between two amino acid sequences or between two nucleotide sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE. Herein, the NEEDLE program from the EMBOSS package was used (version 2.8.0 or higher, EMBOSS: The European Molecular Biology Open Software Suite (2000) Rice,P. Longden,I. and Bleasby,A. Trends in Genetics 16, (6) pp276-277, http://emboss.bioinformatics.nl/). For protein sequences EBLOSUM62 is used for the substitution matrix. For nucleotide sequence, EDNAFULL is used. The optional parameters used are a gap-open penalty of 10 and a gap extension penalty of 0.5. The skilled person will appreciate that all these different parameters will yield slightly different results but that the overall percentage identity of two sequences is not significantly altered when using different algorithms.

After alignment by the program NEEDLE as described above the percentage of sequence identity between a query sequence and a subject sequence is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labelled in the output of the program as "longest-identity".

The disclosed nucleic acid and protein sequences can further be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to nucleic acid molecules as described herein. BLAST protein searches can be performed with the XBLAST program, score = 50, wordlength = 3 to obtain amino acid sequences homologous to protein molecules as described herein. To obtain gapped alignments for comparison purposes, Gapped BLAST can be utilized as described in Altschul et al., (1997) Nucleic Acids Res. 25(17): 3389-3402. When utilizing BLAST and Gapped BLAST programs, the default parameters of the respective programs (e.g., XBLAST and NBLAST) can be used. See the homepage of the National Center for Biotechnology Information at http://www.ncbi.nlm.nih.gov/.

A reference herein to a patent document or other matter which is given as prior art is not to be taken as an admission that that document or matter was known or that the information it contains was part of the common general knowledge as at the priority date of any of the claims.

The invention is further illustrated by the following Examples:

### EXAMPLES

### Example 1: Construction of E. coli expression vectors

The full length open reading frame encoding UGTs from *Solanum lycopersicon* were amplified from S. *lycopersicon* cDNA. 1 µg of total RNA isolated from tomato fruit was used as starting material to prepare cDNA using the SMART™ RACE cDNA Amplification Kit (Clontech), according to the manufacturer's instructions.

For amplification Phusion "proofreading polymerase" (Finnzymes) and the primers mentioned in Table 1 were used.

**Table 1: primers used to amplify tomato and stevia UGT fragments**

| | **Forward primer** | **Reverse primer** |
|---|---|---|
| RT7 | ATTAGGATCCAATGGGAACACAAGTAACAGAG | AATACTGCAGTTAATTAGTACTAATCTTACAAAATTG |
| RT11 | ATTAGGATCCAATGGAAGCCAAGAAAAATAAAATGAG | AATACTGCAGTCATTTGTTGCTGCAAAGAGCCATC |
| RT15 | ATTAGGATCCAATGGATGGTTCGAATGAAAAGTC | AATACTGCAGCTAGACAACATTTGATCTAGTCTTG |
| RT18 | ATTAGGATCCAATGAGTACTACTTTAAAGGTATTGATG | AATACTGCAGATTCACTTATTACTATTCCTACAAAG |
| UGT2_1a | ATTAGGATCCAATGGCCACTTCTGACTCCAT | AATAAAGCTTTTAGCTTTCGTGGTCAATGGCA |
| 85C2 | ATTAGGATCCAATGGACGCTATGGCCACCACT | AATAAAGCTTTTAGTTTCGAGCCAAGACAGTG |

The amplified fragment and vector pACYC-DUET1 (Novagen) were digested with the restriction enzymes *Bam*HI and *Pst*I for the tomato UGT fragments or *Bam*HI and *Hind*III for UGT2_1 a and UGT85C2, followed by purification of the required DNA fragments, their subsequent ligation and finally transformation into *E. coli XL-1* blue using standard procedures. Recombinant bacteria were selected on LB plates containing 50 µg /mL chloramphenicol. After ON growth of recombinant colonies in liquid culture (3 mL LB broth with 50 µg /mL chloramphenicol, 250 rpm, 37°C), plasmid DNA was isolated using the Qiaprep Spin Miniprep kit (Qiagen). Isolated plasmid material was checked by Sanger sequencing with vector primers.

This cloning strategy led to constructs from which the UGTs can be expressed with an N-terminal His₆-tag

### Example 2: Synthesis of Steviolmonoside by UGT85C2

To prepare Steviolmonoside enzymatically from Steviol (Sigma U4625) and UDP-glucose (Sigma 4625), the following compounds were mixed in a total reaction volume of 4 ml. For preparation of a crude enzyme extract of UGT85C2 see Example 3.

| | **µl** |
|---|---|
| 100 mM 2-mercaptoethanol in 0.1M Tris-buffer | 160 |
| 100 mM UDP-glucose in 10% DMSO | 800 |
| 100 mM Steviol in 100% DMSO | 40 |
| Crude enzyme extract UGT85C2 | 400 |

The glycosylation reaction was performed overnight at 30 °C and 100 rpm. Subsequently the reaction was purified using an Oasis hydrophiliclipophilic-Balanced (HLB) 3 cc extraction cartridge (Waters), which had been preconditioned according to the manufacturer's instructions. The enzymatic reaction was loaded on the HLB column, and allowed to enter the column by gravity flow. Subsequently the column was washed with 6 mL of water. Product was eluted by passing 3 ml of 100% methanol over the column. The methanol elute was dried under vacuum centrifugation and the pellet dissolved in 80 µl DMSO. This resulted in a 50 mM steviolmonoside preparation.

### Example 3: In vitro comparison of different tomato UGTs and Stevia UGT2 1a

The control plasmid pACYC-DUET-1 and the UGT constructs were transformed to *E. coli* BL21 DE3 (Invitrogen). For expression, a 3 mL overnight culture of the recombinant *E. coli* strains was prepared (LB medium with appropriate antibiotic; 50 ug chloramphenicol / mL and 1% glucose). 200 µL of that culture was transferred to 20 mL of LB medium with the appropriate antibiotic in a 100 mL Erlenmeijer flask, and incubated at 37°C, 250 rpm until the A600 was 0.4 to 0.6. Subsequently IPTG was added to a final concentration of 1 mM and cultures were incubated overnight at 18°C and 250 rpm. The next day, cells were harvested by centrifugation (10 min 8000xg), medium was removed, and cells were resuspended in 1 mL Resuspension buffer (100 mM Tris-HCI pH = 7.5, 1.4 mM 2-mercaptoethanol; 4°C, 15% glycerol). Cells were disrupted by two times shaking with 200 mg 0.1 mm Zirconia/Silica Beads (BioSpec) for 10 seconds in a FastPrep FP120 machine (Savant) at speed 6.5 . Insoluble particles were subsequently removed by centrifugation (10 min 13,000xg, 4°C). The resulting supernatants were referred to as crude enzyme extracts.

### Example 4: Glucosylation of Steviolmonoside and RebaudiosideA by UGTs

For enzyme assays, a mix of total 50 µl was made in a 2 ml eppendorf tube:

| | |
|---|---|
| 0.1M Tris in 2% DMSO | 37.5µl |
| 100 mM 2-mercaptoethanol in 0.1M tris | 2 µl |
| 100 mM UDP-glucose in 10%DMSO | 5 µl |
| 50 mM Steviolmonoside in 100%DMSO | 0.5 µl |
| Crude UGT enzyme extract | 5 µl |

The tubes were incubated overnight at 30 °C and 100 rpm.

For assays with Rebaudiosise-A (RebA), Steviolmonoside was replaced by 0.5 µl 50mM RebA (ChromDex ASB-00018225) in 50% DMSO.

### Example 5: LC-MS analyses

An LC-PDA-QTOF-MS system was used to analyse reaction products. After incubation, the *in vitro* enzyme assay mix (50 µl) was stopped by addition of 150 µl of 100% methanol in MQ water acidified with 0.13% formic acid. Samples were sonicated for 15 min, centrifuged at 2500 rpm for 10 min and filtered through 0.45µm filters (Minisart SRP4, Biotech GmbH, Germany). For chromatographic separation, a Luna C18(2) pre-column (2.0 x 4 mm) and an analytical column (2.0 x 150 mm, 100Å, particle size 3µm) from Phenomenex (Torrance, CA, USA) were used. Five microliters of each filtered sample were injected into the system for LC-PDA-MS analysis using formic acid : water (1:1000, v/v; eluent A) and formic acid : acetonitrile (1:1000, v/v; eluent B) as elution solvents. Flow was set at 0.19 mL/min with the gradient from 80% eluent A and 20% eluent B to 45% Eluent A and 55% eluent B across a period of 45 min. The column temperature was maintained at 40°C and the samples at 20°C. UV absorbance was measured using a Waters 2996 PDA (A range from 240 to 600 nm) and ESI-MS analysis was performed using a QTOF Ultima V4.00.00 mass spectrometer (Waters-Corporation, MS technologies) in negative mode. A collision energy of 10 eV was used for full-scan LC-MS in the m/z range 100 to 1,500. Leucine enkephalin ([M - H]⁻ = 554.2620), was used for online mass calibration (lock mass).

Compounds were identified by their retention time and their apparent mass and compared to standard steviosides present in the Rebaudioside-A Impurities Mix-6 (Cerilliant S-017) (Table 2).

**Table 2: Retention time and masses of steviosides in the Rebaudioside-A Impurities Mix-6**

| | **Rt (min)** | **m/z** |
|---|---|---|
| RebD | 12.95 | 1127.47 |
| RebA | 18.76 | 965.42 |
| Stevioside | 18.94 | 803.37 |
| Rubusoside | 22.84 | 803.37 |
| RebB | 25.15 | 641.31 |
| Steviolbioside | 25.58 | 641.31 |
| Steviol | 44.73 | 317.21 |

The results of the *in vitro* tests are given in Table 3 and 4. To semi-quantify the produced compounds of the *in vitro* assays, the peak surface area for each relevant peak was measured from the total ion count chromatograms . Clearly, UGT2_1a was able to produce steviolbioside (Rt = 25.6) from Steviolmonoside. UGT RT18 also produces predominantly steviolbioside. Other RTs produce preferentially other steviolglycosides (Table 3).

**Table 3: Products detected by LC-MS after in vitro reaction of Steviolmonoside with different UGT enzymes. As substrate, Steviolmonoside (Rt=30.4 min; m/z 959 =f2M-Hl) was used. Shown are peak surface area in the LC-MS chromatograms. Rt: retention time in minutes. Steviolbioside is detected at 25.6 min.**

| | m/z=1011 | m/z=407 | m/z = 803 | m/z = 803 | m/z = 641 | m/z = 641 | m/z = 641 |
|---|---|---|---|---|---|---|---|
| | Rt=14.0 | Rt=19.0 | Rt=20.7 | Rt=20.8 | Rt=24.8 | Rt=25.6 | Rt=26.3 |
| blanc | | | | | | 2 | |
| UGT2_1a | | 23 | | | | 11532 | |
| RT18 | | | | | 153 | 4408 | |
| RT15 | | | 540 | | 5841 | 8467 | 1 |
| RT11 | 3 | | | 10110 | 16229 | 2163 | |
| RT7 | | | | 163 | 7764 | 1788 | |

When testing RebA as a substrate, it was clear that RT18 showed a relatively strong formation of RebD (Rt=12.9 min) from RebA, in comparison with UGT2_1a, while the other UGTs preferentially produce different RebA-glycosides (Table 4).

**Table 4: Products detected by LC-MS after in vitro reaction of RebA with different UGT enzymes. As substrate, RebA (Rt=18.74 min; m/z 1011=[M-H + formic acid]) was used. Shown are peak surface area in the LC-MS chromatograms. Rt: retention time in minutes. RebD is detected at Rt=12.9 min.**

| | m/z=565 | m/z=1127 | m/z=1127 | m/z=1127 | m/z=1127 | m/z=1127 |
|---|---|---|---|---|---|---|
| | Rt=12.6 | Rt=12.9 | Rt=14.1 | Rt=14.25 | Rt=14.9 | Rt=17.4 |
| Blanc | | 6 | | 12 | | |
| UGT2_1a | | 1037 | 16 | | | |
| RT18 | 98 | 3596 | | 494 | | |
| RT15 | | 376 | | 8946 | 234 | |
| RT11 | | 33 | | 7684 | 159 | |
| RT7 | | 1212 | | 2169 | | 31 |

Thus, RT18 can form steviolbioside from steviolmonoside, and RebD from RebA.

### Example 6: UGT protein content in crude-enzyme extracts

We observed that the activity for the formation of steviolbioside of the RT18 crude enzyme extract was 2-3 fold lower compared to UGT2_1a. To be able to compare the two enzymes for the activity per enzyme molecule in the crude extracts, we analysed the total protein content of the crude enzyme extracts.

First, the extracts were compared for protein content using Protein Dye Reagent concentrate (BIO-RAD 500-0006), according to the manufacturer's instructions, using lyophilized Bovine Serum Albumine BioRad 500-0007) as a standard. Based on this it was observed that UGT2_1 a crude extract contained twice as much protein as the RT18 crude extract (Table 5).

**Table 5: Total protein content of crude enzyme extracts. Protein concentration is given in µg/µl**

| | **Total protein (µg/µl)** |
|---|---|
| pACYC-DUET-1 | 3.35 |
| UGT2_1a | 5.08 |
| RT18 | 2.66 |
| RT15 | 3.63 |
| RT11 | 3.33 |
| RT7 | 2.06 |

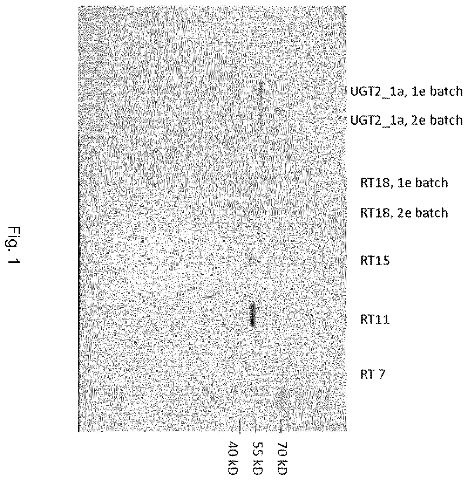

Subsequently, to compare the enzyme concentrations in the crude extracts, a western blot experiment was performed. 50 µg of total protein was brought in 50 ul Sample buffer (20 mM Tris pH 6.8, 6% glycerol, 0.4% SDS, 20 mM Dithiothreitol, 0.01% Bromophenol Blue) and boiled for 5 minutes. Subsequently 10 µl sample (= 10 µg total protein) was loaded on a 12.5 % poly-acryl amide gel with SDS and run for 2 hours at 20 mA. Proteins were transferred from the gel onto nitrocellulose membrane (BIO-RAD) in standard blotting buffer (3 g/L Tris, 14.4 g/L glycine, 10% ethanol) for 1 hour (100 V). The nitrocellulose was subsequently washed with TBST buffer (20 mM Tris-CI buffer pH 7.5, 150 mM NaCl, 0.05% Tween 20) for 5 minutes, and blocked with TBST buffer with 2% non-fat dry milk powder (ELK) for 1 hour. The presence of enzyme was detected by incubation for 1 hour with TBST with 2% ELK and 1:4000 diluted antiHis monoclonal antibody conjugated to peroxidase (Sigma, St Louis, A7058). After washing four times five minutes with TBST, the peroxidase was detected by the TMB Liquid substrate system for membranes (Sigma T0565). A purple colour was detected at the position where His-tagged proteins (here: UGTs) were present on the blot. When all five crude enzyme extracts were compared in this way (Fig. 1) it was clear that UGT2_1a was expressed to well-detectable levels, while RT18 protein could not be detected. The other UGTs (RT15, RT11,RT7) were also detected, to different intensities.

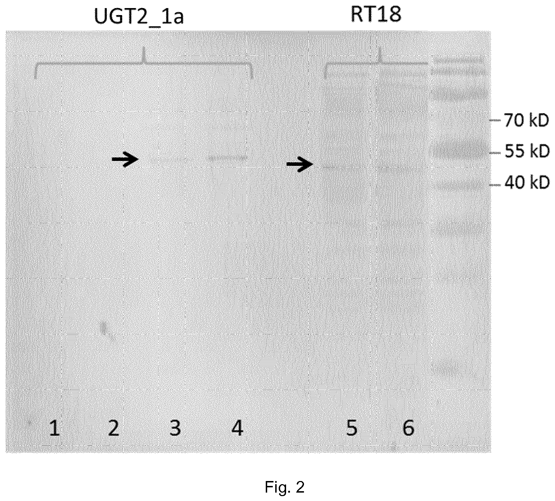

To compare the UGT content in the crude enzyme extracts of RT18 and UGT2_1a, another western blot was made. For UGT2_1a, 0.5, 1.0, 1.9, 3.8 µg protein was loaded, while for RT18, 31.9 and 63.8 µg was loaded. Detection of UGTs was performed as described above. The blot (Fig. 2) showed that the amount of His-tagged UGT protein was the same in 1.9 µg UGT2_1a extract and 63.8 µg RT18, as estimated by visual inspection. This indicated that the concentration of UGT protein in the RT18 crude extract was 20-50 fold lower than in the UGT2_1a crude extract. Thus, the activity of RT18, as recorded in Tables 3 and 4, is more than 10-fold higher than UGT2_1a when using steviolmonoside as a substrate, and more than 50-fold higher when using RebA as a substrate.

### Example 7: MSMS analysis

To provide more evidence that the products of UGT2_1a and RT18 with steviolmonoside as a substrate were identical, the steviolbioside product of RT18 was further compared to the steviolbioside product from UGT2_1a and the steviol-diglucoside product from RT11 by tandem mass spectrometry analysis (LC-MS²). The methanol extracts from the RT18 and UGT2_1a enzyme assays were injected in an Accela HPLC-PDA (Thermo) coupled to a LTQ Ion Trap-Orbitrap FTMS hybrid mass spectrometer (Thermo) system was used. Data-directed MSMS was performed using the same LC conditions as described for LC-QTOF MS analysis (see above), and using negative ionization mode, with an Isolation Width of 3.00 Dalton and a Normalized Collission Energy of 35.0. Retention times of steviolglucosides differed slightly from the analysis on the LC-QTOF MS system (see above, Table 3).

Fragmentation was performed on the compounds with m/z 641.30 eluting at 23.0 min in the RT18 and UGT2_1a samples and eluting at 22.2 min in the RT11 sample.
In the fragmentation spectra of ions of m/z 641.30, the fragments m/z 479.26 [M-H-Glucose] and m/z 317.21 [M-H-2Glucose] were observed in all three samples. The ratio between the m/z 317.21 and m/z 479.26 ions was recorded for all three compounds. For both the RT18 and UGT2_1a compounds, the ratio m/z 317 to m/z 479 was 2:10, while the the ratio m/z 317 to m/z 479 for the RT11 compound was 4:10. Thus, the MS2 analysis did not differentiate the steviolbioside products from RT18 and UGT2_1a, but did differentiate the RT11 steviol diglucoside product from these two. These results further confirm that the major product of RT18 corresponds to steviolbioside.

### Example 8. RT18 expression in steviol glycoside production strain

In order to demonstrate the effect of the in vivo activity of the RT18 enzyme on the production of steviol glycosides, RT18 (SEQ ID NO: 19) was assembled with three promoters of different strength (Table 6), and transformed to a *Yarrowia lipolytica* strain that produces steviol glycosides using the approach described in WO2013/110673 and WO2015/007748. The genotype of this Yarrowia strain is given in Table 7

**Table 6. Different strength promotors used for RT18 expression**

| **Relative promoters strength** | **Name** |
|---|---|
| Weak | CWP (SEQ ID NO: 20) |
| Medium | SCP2 (SEQ ID NO: 21) |
| Strong | HSP (SEQ ID NO: 22) |

**Table 7. Genotype of parental strain (copy number; SEQ ID NO).**

| | |
|---|---|
| Parent strain genotype | MATB tHMG (2; SEQ ID NO: 23) GGS (3; SEQ ID NO: 24) CPS (5; SEQ ID NO: 25) KS (4; SEQ ID NO: 26) KO (3; SEQ ID NO: 27) KAH4 (4; SEQ ID NO: 28) CPR (2; SEQ ID NO: 29) UGT1 (3; SEQ ID NO: 30) UGT2 (2; SEQ ID NO: 31) UGT3 (2; SEQ ID NO: 32) UGT4 (3; SEQ ID NO: 33) |

For positive transformants, a pre-culture was inoculated with colony material from YEPh-D agar. The pre-culture was grown in 200 µl YEP with glucose as carbon source. The pre-culture was incubated 72 hours in an Infors incubator at 30°C, 750 rpm and 80% humidity. 40 µl of pre-culture was used to inoculate 2.5 ml main culture. The main cultures were incubated 120 hours in an Infors incubator at 30°C, 550 rpm, 80% humidity. After 120 h the main culture was spun down at 2750 rpm for 10 min. Supernatant was diluted with water and acetonitrile, and measured using LC/MS.

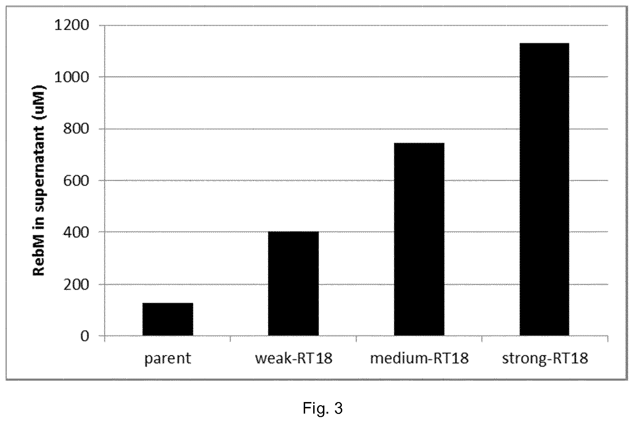

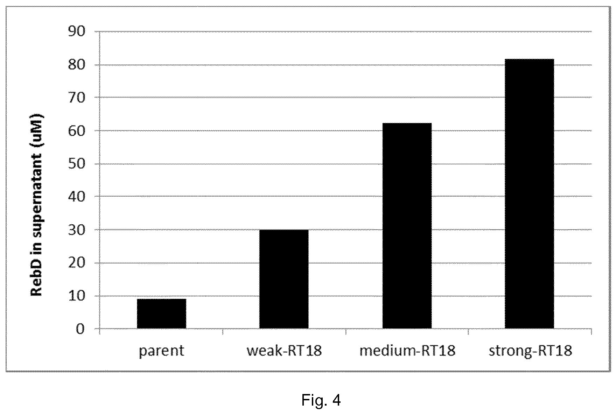

The results are set out in in Figures 3 and 4. It can be seen that the strains that express the RT18 produce higher amounts of RebM and RebD compared to the parent. In addition, the stronger the expression, the more RebD and RebM were produced. The formation of higher RebD illustrates RT18 is effective in catalyzing the glycosylation of the glucose on the 19-position of steviol glycosides (see Figure 6), for example catalyzing the formation of RebD from RebA. RebD can then be further converted to RebM, catalyzed by UGT4.

### Example 9. RT18 and UGT4 expression in steviol glycoside production strain

The expression of other UDP-glycosyl transferases, in combination with RT18, will have an influence on the product profile. For example the RebD that is over-produced in a strain expressing RT18 can be further converted to RebM by the activity of UGT4. In order to evaluate the effect of over-expression of RT18 with UGT4, expression vectors of RT18 and UGT4 were transformed to a *Yarrowia lipoitica* strain producing steviol glycosides using the approach described in WO2013/110673 and WO2015/007748. The genotype of this parental strain is given in Table 8.

**Table 8. Genotype of strain used to transform RT18 and UGT4 (copy number; SEQ ID NO)**

| | |
|---|---|
| Parent strain genotype | MATB tHMG (2; SEQ ID NO: 23) GGS (2; SEQ ID NO: 24) CPS (2; SEQ ID NO: 25) KS (2; SEQ ID NO: 26) KO (2; SEQ ID NO: 27) KAH4 (2; SEQ ID NO: 28) CPR (2; SEQ ID NO: 29) UGT1 (2; SEQ ID NO: 30) UGT2 (1; SEQ ID NO: 34) UGT3 (2; SEQ ID NO: 32) UGT4 (2; SEQ ID NO: 33) |

For positive transformants, a pre-culture was inoculated with colony material from YEPh-D agar. The pre-culture was grown in 200 µl YEP with glucose as carbon source. The pre-culture was incubated 72 hours in an Infors incubator at 30°C, 750 rpm and 80% humidity. 40 µl of pre-culture was used to inoculate 2.5 ml main culture. The main cultures were incubated 120 hours in an Infors incubator at 30°C, 550 rpm, 80% humidity. After 120 h the main culture was spun down at 2750 rpm for 10 min. Supernatant was diluted with water and acetonitrile, and measured using LC/MS.

The results are set out in Table 9, where the percentages of steviol glycosides on total steviol glycosides are listed for the two strains. It can be seen that the strains that expresses the RT18 in combination with additional UGT4 effectively convert a higher percentage of the steviol glycosides to higher glycosylated steviol glycosides. Particularly, RebB, Stevioside and RebA are lower in the strain expressing the RT18 and UGT4, whereas the abundance of RebM is greatly increased. This illustrates the effectiveness of RT18 expression in steering steviol glycoside production towards higher glycosylated products such as RebM.

**Table 9. Percentages of steviol glycosides of total steviol glycosides in parent strain and strain expressing RT18 and an extra copy of UGT4.**

| **Strain** | **RebM** | **RebD** | **RebA** | **Stevioside** | **RebB** | **Other steviol glycosies** |
|---|---|---|---|---|---|---|
| parent | 3 | 6 | 54 | 25 | 7 | 6 |
| RT18, UGT4 | 66 | 5 | 20 | 1 | 1 | 6 |

**Table 10: Description of the sequence listing**

| **SEQ ID NO** | **Description** |
|---|---|
| SEQ ID NO: 1 | amino acid sequence of the RT7 protein from *Solanum lycopersicon* (Solyc11g007480 - tomato genome: http://solgenomics.net/) |
| SEQ ID NO: 2 | amino acid sequence of the RT11 protein from *Solanum lycopersicon* (Solyc11g007500) |
| SEQ ID NO: 3 | amino acid sequence of the RT15 protein from *Solanum lycopersicon* (Solyc04g081830) |
| SEQ ID NO: 4 | amino acid sequence of the RT18 protein from *Solanum lycopersicon* (Solyc05g005930) |
| SEQ ID NO: 5 | amino acid sequence of the UGT2_1a protein from *Stevia rebaudiana* |
| SEQ ID NO: 6 | amino acid sequence of the UGT85C2 protein from *Stevia rebaudiana* |
| SEQ ID NO: 7 | nucleic acid sequence of the RT7 forward primer |
| SEQ ID NO: 8 | nucleic acid sequence of the RT7 reverse primer |
| SEQ ID NO: 9 | nucleic acid sequence of the RT11 forward primer |
| SEQ ID NO: 10 | nucleic acid sequence of the RT11 reverse primer |
| SEQ ID NO: 11 | nucleic acid sequence of the RT15 forward primer |
| SEQ ID NO: 12 | nucleic acid sequence of the RT15 reverse primer |
| SEQ ID NO: 13 | nucleic acid sequence of the RT18 forward primer |
| SEQ ID NO: 14 | nucleic acid sequence of the RT18 reverse primer |
| SEQ ID NO: 15 | nucleic acid sequence of the UGT2_1a forward primer |
| SEQ ID NO: 16 | nucleic acid sequence of the UGT2_1a reverse primer |
| SEQ ID NO: 17 | nucleic acid sequence of the UGT85C2 forward primer |
| SEQ ID NO: 18 | nucleic acid sequence of the UGT82C2 reverse primer |
| SEQ ID NO: 19 | nucleic acid sequence of the RT18 open reading frame optimized for expression in *Y. lipolytica* |
| SEQ ID NO: 20 | nucleic acid sequence of CWP promoter from *Y. lipolytica* |
| SEQ ID NO: 21 | nucleic acid sequence of SCP2 promoter from *Y. lipolytica* |
| SEQ ID NO: 22 | nucleic acid sequence of HSP promoter from *Y. lipolytica* |
| SEQ ID NO: 23 | nucleic acid sequence of tHMG optimized for expression in *Y. lipolitica* |
| SEQ ID NO: 24 | nucleic acid sequence of GGS optimized for expression in *Y. lipolytica* |
| SEQ ID NO: 25 | nucleic sequence of CPS from S. *rebaudiana* optimized for expression in *Y. lipolytica* |
| SEQ ID NO: 26 | nucleic acid sequence of tKS from S. rebaudiana optimized for expression in *Y. lipolitica* |
| SEQ ID NO: 27 | nucleic acid sequence of KO from *Gibberella fujikori* optimized for expression in *Y. lipolytica* |
| SEQ ID NO: 28 | nucleic acid sequence of KAH_4 optimized for expression in *Y. lipolytica* |
| SEQ ID NO: 29 | nucleic acid sequence of CPR_optimized for expression in *Y. lipolytica* |
| SEQ ID NO: 30 | nucleic acid sequence of UGT1 optimized for expression in *Y. lipolytica* |
| SEQ ID NO: 31 | nucleic acid sequence of UGT2 variant optimized for expression in *Y. lipolytica* |
| SEQ ID NO: 32 | nucleic acid sequence of UGT3 optimized for expression in *Y. lipolytica* |
| SEQ ID NO: 33 | nucleic acid sequence of UGT4 optimized for expression in *Y. lipolytica* |
| SEQ ID NO: 34 | nucleic acid sequence of UGT2 variant optimized for expression in *Y. lipolytica* |

## Claims

1. A recombinant host capable of producing a glycosylated diterpene, such as a steviol glycoside, comprising a recombinant nucleic acid sequence encoding a polypeptide comprising the amino acid sequence set forth in SEQ ID NO: 4 or an amino acid sequence having at least 90% sequence identity thereto, wherein the polypeptide has improved UGT2 activity compared to a polypeptide with the amino acid sequence as set forward in SEQ ID NO: 5 and wherein the improved UGT2 activity results in the production of a higher amount of a steviol glycoside.

2. A recombinant host according to claim 1 which comprises one or more recombinant nucleotide sequence(s) encoding:
a polypeptide having ent-copalyl pyrophosphate synthase activity;
a polypeptide having ent-Kaurene synthase activity;
a polypeptide having ent-Kaurene oxidase activity; and
a polypeptide having kaurenoic acid 13-hydroxylase activity.

3. A recombinant host according to any one of the preceding claims, which comprises a recombinant nucleic acid sequence encoding a polypeptide having NADPH-cytochrome p450 reductase activity.

4. A recombinant host according to any one of the preceding claims which comprises a recombinant nucleic acid sequence encoding one or more of:
(i) a polypeptide having UGT74G1 activity;
(ii) a polypeptide having UGT85C2 activity; and
(iii) a polypeptide having UGT76G1 activity.

5. A recombinant host according to any one of the preceding claims which comprises a recombinant nucleic acid sequence encoding an additional polypeptide having UGT2 activity, wherein a polypeptide having UGT2 activity is a polypeptide which has glycosyltransferase activity (EC 2.4) and can act as a catalyst for the transfer of a monosaccharide unit from a glycosyl donor to a glycosyl acceptor molecule, wherein the glycosyl donor is uracil-diphosphate glucose and the glycosyl acceptor molecule is one or more of:
a. steviol-13-O-glucoside, wherein a glucose moiety is transferred to the C-2'of the 13-0-glucose of said steviol-13-O-glucoside to produces steviolbioside; and/or
b. rubusoside, wherein a glucose moiety is transferred the C-2' of the 13-O-glucose of rubusoside to produce stevioside; and/or
c. stevioside, wherein a glucose moiety is transferred to the C-2' of the 19-O-glucose residue to produce rebaudioside E; and/or
d. rebaudioside A, wherein a glucose moiety is transferred to the C-2' of the 19-O-glucose residue to produce rebaudioside D; and/or
e. steviol-19-glucoside, wherein a glucose moiety is transferred to the 19-position to produce steviol-19-2side; and/or
f. rubusoside, wherein a glucose moiety is transferred to the 19-position to produce 13-[(β-D-Glucopyranosyl)oxy)kaur-16-en-18-oic acid 2-O-β-D-glucopyranosyl-β-D-glucopyranosyl ester; and/or
wherein the polypeptide having UGT2 activity can act as a uridine 5'-diphospho D-xylosyl:steviol-13-0-glucoside transferase, transferring a xylose moiety to the C-2' of the 13-0-glucose of steviol-13-O-glucoside; and/or
wherein the polypeptide having UGT2 activity may act as a uridine 5'-diphospho L-rhamnosyl:steviol-13-O-glucoside transferase, transferring a rhamnose moiety to the C-2' of the 13-O-glucose of steviol,
wherein the polypeptide having UGT2 activity does not have the ability to transfer a glucose moiety to steviol compounds having a 1,3-bound glucose at the C-13 position.

6. A recombinant host according to any one of the preceding claims, wherein the host belongs to one of the genera *Saccharomyces, Aspergillus, Pichia, Kluyveromyces, Candida, Hansenula, Humicola, Issatchenkia, Trichosporon, Brettanomyces, Pachysolen, Yarrowia, Yamadazyma* or *Escherichia,* preferably wherein the recombinant host is a Saccharomyces cerevisiae cell, a *Yarrowia lipolytica* cell, a *Candida krusei* cell, an *Issatchenkia orientalis* or an *Escherichia coli* cell.

7. A recombinant host according to any one of the preceding claims, wherein the ability of the host to produce geranylgeranyl diphosphate (GGPP) is upregulated.

8. A recombinant host according to any one of the preceding claims, comprising one or more recombinant nucleic acid sequence(s) encoding hydroxymethylglutaryl-CoA reductase, farnesyl-pyrophosphate synthetase and geranylgeranyl diphosphate synthase.

9. A recombinant host according to any one of the preceding claims which comprises a nucleic acid sequence encoding one or more of:
a polypeptide having hydroxymethylglutaryl-CoA reductase activity;
a polypeptide having farnesyl-pyrophosphate synthetase activity;
a polypeptide having geranylgeranyl diphosphate synthase activity.

10. A process for the preparation of a glycosylated diterpene which comprises fermenting a recombinant host according to any one of claims 1 to 9 in a suitable fermentation medium, and optionally recovering the glycosylated diterpene.

11. A process according to claim 10 for the preparation of a glycosylated diterpene, wherein the process is carried out on an industrial scale.

12. A fermentation broth comprising a recombinant cell according to any one of claims 1 to 9.

13. A method for converting a first glycosylated diterpene into a second glycosylated diterpene, which method comprises:
- contacting said first glycosylated diterpene with a recombinant host according to any one of claims 1 to 9, a cell free extract derived from such a recombinant host or an enzyme preparation derived from either thereof, preferably wherein the first glycosylated diterpene is steviol-13-monoside, steviol-19-monoside, rubusoside, stevioside, Rebaudioside A or 13-[(β-D-Gtucopyranosyt)oxy)kaur-16-en-18-oic acid 2-O-β-D-glucopyranosyl-β-D-glucopyranosyl ester and the second glycosylated diterpene is steviol-19-diside, steviolbioside, stevioside, 13-[(β-D-Gtucopyranosyt)oxy)kaur-16-en-18-oic acid 2-O-β-D-glucopyranosyl-β-D-glucopyranosyl ester, RebE or RebD;
- thereby to convert the first glycosylated diterpene into the second glycosylated diterpene, preferably wherein the second glycosylated diterpene is: steviol-19-diside, steviolbioside, stevioside, 13-[(β-D-Glucopyranosyl)oxy)kaur-16-en-18-oic acid 2-O-β-D-glucopyranosyl-β-D-glucopyranosyl ester, RebE or RebD.

## Patentansprüche

1. Rekombinanter Wirt mit der Fähigkeit zur Produktion eines glykosylierten Diterpens, wie z. B. eines Steviolglycosids, umfassend eine rekombinante Nukleinsäuresequenz, die ein die Aminosäuresequenz gemäß SEQ ID NO: 4 oder eine Aminosäuresequenz mit einer Sequenzidentität von wenigstens 90% damit umfassendes Polypeptid codiert, wobei das Polypeptid verbesserte UGT2-Aktivität verglichen mit einem Polypeptid mit der Aminosäuresequenz gemäß SEQ ID NO: 5 aufweist und wobei die verbesserte UGT2-Aktivität zur Produktion einer größeren Menge eines Steviolglycosids führt.

2. Rekombinanter Wirt nach Anspruch 1, der eine oder mehrere rekombinante Nukleotidsequenz(en) umfasst, die Folgendes codiert bzw. codieren:
ein Polypeptid mit *ent*-Copalylpyrophosphatsynthase-Aktivität;
ein Polypeptid mit *ent-*Kaurensynthase-Aktivität;
ein Polypeptid mit ent-Kaurenoxidase-Aktivität; und
ein Polypeptid mit Kaurensäure-13-hydroxylase-Aktivität.

3. Rekombinanter Wirt nach einem der vorhergehenden Ansprüche, der eine rekombinante Nukleinsäuresequenz umfasst, die ein Polypeptid mit NADPH-Cytochrom-p450-Reduktase-Aktivität codiert.

4. Rekombinanter Wirt nach einem der vorhergehenden Ansprüche, der eine rekombinante Nukleinsäuresequenz umfasst, die ein oder mehrere der folgenden codiert:
(i) ein Polypeptid mit UGT74G1-Aktivität;
(ii) ein Polypeptid mit UGT85C2-Aktivität; und
(iii) ein Polypeptid mit UGT76G1-Aktivität.

5. Rekombinanter Wirt nach einem der vorhergehenden Ansprüche, der eine rekombinante Nukleinsäuresequenz umfasst, die ein zusätzliches Polypeptid mit UGT2-Aktivität codiert, wobei es sich bei einem Polypeptid mit UGT2-Aktivität um ein Polypeptid handelt, das Glycosyltransferase-Aktivität aufweist (EC 2.4) und als Katalysator für die Übertragung einer Monosaccharideinheit von einem Glycosyldonorauf ein Glycosylakzeptormolekül fungieren kann, wobei es sich bei dem Glycosyldonor um Uracildiphosphatglucose und bei dem Glycosylakzeptormolekül um ein oder mehrere der folgenden handelt:
a. Steviol-13-0-glucosid, wobei eine Glucosegruppierung auf das C-2' der 13-O-Glucose des Steviol-13-0-glucosids unter Erhalt von Steviolbiosid übertragen wird; und/oder
b. Rubusosid, wobei eine Glucosegruppierung auf das C-2' der 13-O-Glucose von Rubusosid unter Erhalt von Steviosid übertragen wird; und/oder
c. Steviosid, wobei eine Glucosegruppierung auf das C-2' des 19-O-Glucoserests unter Erhalt von Rebaudiosid E übertragen wird; und/oder
d. Rebaudiosid A, wobei eine Glucosegruppierung auf das C-2' des 19-O-Glucoserests unter Erhalt von Rebaudiosid D übertragen wird; und/oder
e. Steviol-19-glucosid, wobei eine Glucosegruppierung auf die 19-Position unter Erhalt von Steviol-19-2sid übertragen wird; und/oder
f. Rubusosid, wobei eine Glucosegruppierung auf die 19-Position unter Erhalt von 13-[(β-D-Glucopyranosyl)oxy)kaur-16-en-18-säure 2-O-β-D-glucopyranosyl-β-D-glucopyranosylester übertragen wird; und/oder
wobei das Polypeptid mit UGT2-Aktivität als Uridin-5'-diphospho-D-xylosyl:Steviol-13-O-glucosid-Transferase fungieren kann, wobei eine Xylosegruppierung auf das C-2' der 13-O-Glucose von Steviol-13-0-glucosid übertragen wird; und/oder wobei das Polypeptid mit UGT2-Aktivität möglicherweise als Uridin-5'-diphospho-L-rhamnosyl:Steviol-13-O-glucosid-Transferase fungiert, wobei eine Rhamnosegruppierung auf das C-2' der 13-O-Glucose von Steviol übertragen wird,
wobei das Polypeptid mit UGT2-Aktivität nicht in der Lage ist, eine Glucosegruppierung auf Steviolverbindungen mit einer 1,3-gebundenen Glucose an der C-13-Position zu übertragen.

6. Rekombinanter Wirt nach einem der vorhergehenden Ansprüche, wobei der Wirt zu einem der Genera *Saccharomyces, Aspergillus, Pichia, Kluyveromyces, Candida, Hansenula, Humicola, Issatchenkia, Trichosporon, Brettanomyces, Pachysolen, Yarrowia, Yamadazyma* oder *Escherichia* gehört, vorzugsweise wobei es sich bei dem rekombinanten Wirt um eine *Saccharomyces cerevisiae-Zelle,* eine *Yarrowia lipolytica-Zelle,* eine *Candida* krusei-Zelle, eine *Issatchenkia orientalis-Zelle* oder eine *Escherichia* coli-Zelle handelt.

7. Rekombinanter Wirt nach einem der vorhergehenden Ansprüche, wobei das Vermögen des Wirts, Geranylgeranyldiphosphat (GGPP) zu produzieren, heraufreguliert ist.

8. Rekombinanter Wirt nach einem der vorhergehenden Ansprüche, umfassend eine oder mehrere rekombinante Nukleotidsequenz(en), die Hydroxymethylglutaryl-CoA-Reduktase, Farnesylpyrophosphat-Synthetase und Geranylgeranyldiphosphat-Synthase codieren.

9. Rekombinanter Wirt nach einem der vorhergehenden Ansprüche, der eine Nukleinsäuresequenz umfasst, die ein oder mehrere der folgenden codiert:
ein Polypeptid mit Hydroxymethylglutaryl-CoA-Reduktase-Aktivität;
ein Polypeptid mit Farnesylpyrophosphat-Synthetase-Aktivität;
ein Polypeptid mit Geranylgeranyldiphosphat-Synthase-Aktivität.

10. Verfahren zur Herstellung eines glykosylierten Diterpens, das Fermentieren eines rekombinanten Wirts nach einem der Ansprüche 1 bis 9 in einem geeigneten Fermentationsmedium und gegebenenfalls Gewinnen des glykosylierten Diterpens umfasst.

11. Verfahren nach Anspruch 10 zur Herstellung eines glykosylierten Diterpens, wobei das Verfahren im großtechnischen Maßstab durchgeführt wird.

12. Fermentationsbrühe, umfassend eine rekombinante Zelle nach einem der Ansprüche 1 bis 9.

13. Methode zur Überführung eines ersten glykosylierten Diterpens in ein zweites glykosyliertes Diterpen, wobei die Methode Folgendes umfasst:
- Inkontaktbringen des ersten glykosylierten Diterpens mit einem rekombinanten Wirt nach einem der Ansprüche 1 bis 9, einem von einem solchen rekombinanten Wirt stammenden zellfreien Extrakt oder einer von einem dieser beiden stammenden Enzympräparation, vorzugsweise wobei es sich bei dem ersten glykosylierten Diterpen um Steviol-13-monosid, Steviol-19-monosid, Rubusosid, Steviosid, Rebaudiosid A oder 13-[(β-D-Glucopyranosyl)-oxy)kaur-16-en-18-säure-2-O-β-D-glucopyranosyl-β-D-glucopyranosylester und bei dem zweiten glykosylierten Diterpen um Steviol-19-disid, Steviolbiosid, Steviosid, 13-[(β-D-Glucopyranosyl)oxy)kaur-16-en-18-säure-2-O-β-D-glucopyranosyl-β-D-glucopyranosylester, RebE oder RebD handelt;
- dadurch das erste glykosylierte Diterpen in das zweite glykosylierte Diterpen zu überführen, vorzugsweise wobei es sich bei dem zweiten glykosylierten Diterpen um Folgendes handelt: Steviol-19-disid, Steviolbiosid, Steviosid, 13-[(β-D-Glucopyranosyl)oxy)kaur-16-en-18-säure-2-O-β-D-glucopyranosyl-β-D-glucopyranosylester, RebE oder RebD.

## Revendications

1. Hôte recombinant capable de produire un diterpène glycosylé, tel qu'un glycoside de stéviol, comprenant une séquence d'acides nucléiques recombinante codant pour un polypeptide qui comprend la séquence d'acides aminés indiquée dans la SEQ ID NO: 4 ou une séquence d'acides aminés ayant une identité de séquence d'au moins 90% avec celle-ci, dans lequel le polypeptide a une activité d'UGT2 améliorée en comparaison avec un polypeptide avec la séquence d'acides aminés telle que présentée dans la SEQ ID NO: 5, et dans lequel l'activité d'UGT2 améliorée conduit à la production d'une quantité plus élevée d'un glycoside de stéviol.

2. Hôte recombinant selon la revendication 1, qui comprend une ou plusieurs séquence(s) nucléotidique(s) recombinante(s) codant pour :
un polypeptide ayant une activité d'ent-copalyl pyrophosphate synthase ;
un polypeptide ayant une activité d'ent-kaurène synthase ;
un polypeptide ayant une activité d'ent-kaurène oxydase ; et
un polypeptide ayant une activité de 13-hydroxylase de l'acide kaurénoïque.

3. Hôte recombinant selon l'une quelconque des revendications précédentes, qui comprend une séquence d'acides nucléiques recombinante codant pour un polypeptide ayant une activité de NADPH-cytochrome p450 réductase.

4. Hôte recombinant selon l'une quelconque des revendications précédentes, qui comprend une séquence d'acides nucléiques recombinante codant pour un ou plusieurs élément(s) parmi :
(i) un polypeptide ayant une activité d'UGT74G1 ;
(ii) un polypeptide ayant une activité d'UGT85C2 ; et
(iii) un polypeptide ayant une activité d'UGT76G1.

5. Hôte recombinant selon l'une quelconque des revendications précédentes, qui comprend une séquence d'acides nucléiques recombinante codant pour un polypeptide supplémentaire ayant une activité d'UGT2, dans lequel un polypeptide ayant une activité d'UGT2 est un polypeptide qui a une activité de glycosyltransférase (EC 2.4) et qui peut agir comme catalyseur pour le transfert d'une unité monosaccharidique à partir d'un donneur de glycosyle vers une molécule accepteur de glycosyle, dans lequel le donneur de glycosyle est l'uracile-diphosphate glucose et la molécule accepteur de glycosyle est un ou plusieurs élément(s) parmi :
a. le stéviol-13-0-glucoside, dans lequel une fraction de glucose est transférée vers le C-2' du 13-0-glucose dudit stéviol-13-0-glucoside pour produire le stéviolbioside; et/ou
b. le rubusoside, dans lequel une fraction de glucose est transférée vers le C-2' du 13-O-glucose du rubusoside pour produire le stévioside ; et/ou
c. le stévioside, dans lequel une fraction de glucose est transférée vers le C-2' du résidu de 19-O-glucose pour produire le rébaudioside E ; et/ou
d. le rébaudioside A, dans lequel une fraction de glucose est transférée vers le C-2' du résidu de 19-O-glucose pour produire le rébaudioside D ; et/ou
e. un stéviol-19-glucoside, dans lequel une fraction de glucose est transférée vers la position 19 pour produire un stéviol-19-2side ; et/ou
f. le rubusoside, dans lequel une fraction de glucose est transférée vers la position 19 pour produire le 2-0-β-D-glucopyranosyl-β-D-glucopyranosyl ester de l'acide 13-[(β-D-glucopyranosyl)oxy)kaur-16-en-18-oïque ; et/ou
dans lequel le polypeptide ayant une activité d'UGT2 peut agir comme une uridine 5'-diphospho D-xylosyl:stéviol-13-O-glucoside transférase, en transférant une fraction de xylose vers le C-2' du 13-O-glucose d'un stéviol-13-O-glucoside ; et/ou
dans lequel le polypeptide ayant une activité d'UGT2 peut agir comme une uridine 5'-diphospho L-rhamnosyl:stéviol-13-O-glucoside transférase, en transférant une fraction de rhamnose vers le C-2' du 13-O-glucose du stéviol, dans lequel le polypeptide ayant une activité d'UGT2 n'a pas la faculté de transférer une fraction de glucose vers des composés de stéviol ayant un glucose lié en 1,3 au niveau de la position C-13.

6. Hôte recombinant selon l'une quelconque des revendications précédentes, l'hôte appartenant à l'un des genres *Saccharomyces, Aspergillus, Pichia, Kluyveromyces, Candida, Hansenula, Humicola, Issatchenkia, Trichosporon, Brettanomyces, Pachysolen, Yarrowia, Yamadazyma* ou *Escherichia,* de préférence l'hôte recombinant étant une cellule de *Saccharomyces cerevisiae,* une cellule de *Yarrowia lipolytica,* une cellule de *Candida krusei,* une cellule *d'Issatchenkia orientalis* ou une cellule *d'Escherichia coli.*

7. Hôte recombinant selon l'une quelconque des revendications précédentes, dans lequel la faculté de l'hôte à produire du géranylgéranyl diphosphate (GGPP) est régulée à la hausse.

8. Hôte recombinant selon l'une quelconque des revendications précédentes, comprenant une ou plusieurs séquence(s) d'acides nucléiques recombinante(s) codant pour une hydroxyméthylglutaryl-CoA réductase, une farnésyl-pyrophosphate synthétase et une géranylgéranyl diphosphate synthase.

9. Hôte recombinant selon l'une quelconque des revendications précédentes, qui comprend une séquence d'acides nucléiques codant pour un ou plusieurs élément(s) parmi :
un polypeptide ayant une activité d'hydroxyméthylglutaryl-CoA réductase ;
un polypeptide ayant une activité de farnésyl-pyrophosphate synthétase ;
un polypeptide ayant une activité de géranylgéranyl diphosphate synthase.

10. Processus destiné à la préparation d'un diterpène glycosylé qui comprend les étapes consistant à faire fermenter un hôte recombinant, selon l'une quelconque des revendications 1 à 9, dans un milieu de fermentation approprié et, en option, à récupérer le diterpène glycosylé.

11. Processus, selon la revendication 10, pour la préparation d'un diterpène glycosylé, le processus étant effectué à une échelle industrielle.

12. Bouillon de fermentation comprenant une cellule recombinante selon l'une quelconque des revendications 1 à 9.

13. Procédé destiné à convertir un premier diterpène glycosylé en un deuxième diterpène glycosylé, lequel procédé comprend les étapes consistant à :
- mettre en contact ledit premier diterpène glycosylé avec un hôte recombinant selon l'une quelconque des revendications 1 à 9, un extrait acellulaire dérivé d'un tel hôte recombinant ou une préparation enzymatique dérivée de l'un ou l'autre de ceux-ci, de préférence dans lequel le premier diterpène glycosylé est un stéviol-13-monoside, un stéviol-19-monoside, le rubusoside, le stévioside, le rébaudioside A ou le 2-0-β-D-glucopyranosyl-β-D-glucopyranosyl ester de l'acide 13-[(β-B-glucopyranosyl)oxy)kaur-16-en-18-oïque et le deuxième diterpène glycosylé est un stéviol-19-diside, le stéviolbioside, le stévioside, le 2-O-β-D-glucopyranosyl-β-D-glucopyranosyl ester de l'acide 13-[(β-D-glucopyranosyl)oxy)kaur-16-en-18-oïque, le RebE ou le RebD ;
- pour convertir de ce fait le premier diterpène glycosylé en le deuxième diterpène glycosylé, de préférence dans lequel le deuxième diterpène glycosylé est : un stéviol-19-diside, le stéviolbioside, le stévioside, le 2-O-β-D-glucopyranosyl-β-D-glucopyranosyl ester de l'acide 13-[(β-D-glucopyranosyl)oxy)kaur-16-en-18-oïque, le RebE ou le RebD.


### SEQUENCE LISTING

<110> DSM IP Assets B.V.
<120> UDP-glycosyltransferases
<130> 30028-WO-PCT
<150> US62/136759
   <151> 2015-03-23
<160> 34
<170> PatentIn version 3.5
<210> 1
   <211> 453
   <212> PRT
   <213> Solanum lycopersicon
<400> 1
   Met Gly Thr Gln Val Thr Glu His Gly Thr Ser Asn Leu Arg Val Val
   1               5                   10                  15
   Met Phe Pro Trp Leu Ala Tyr Gly His Ile Ser Pro Phe Leu Tyr Val
               20                  25                  30
   Ala Lys Lys Leu Ala Asp Arg Gly Phe Leu Ile Tyr Leu Cys Ser Thr
           35                  40                  45
   Pro Ile Asn Leu Lys Ser Thr Ile Glu Lys Ile Pro Glu Lys Tyr Ala
       50                  55                  60
   Asp Ser Ile His Leu Ile Glu Leu His Leu Pro Glu Leu Pro Glu Leu
   65                  70                  75                  80
   Pro Pro His Tyr His Thr Thr Asn Gly Leu Pro Pro His Leu Asn His
                   85                  90                  95
   Thr Leu Gln Lys Ala Leu Lys Met Ser Lys Pro Asn Leu Ser Lys Ile
               100                 105                 110
   Leu Lys Asn Leu Lys Pro Asp Leu Met Ile Tyr Asp Val Leu Gln Gln
           115                 120                 125
   Trp Ala Glu Arg Val Ala Asn Glu Gln Ser Ile Pro Ala Val Arg Leu
       130                 135                 140
   Leu Thr Phe Gly Ala Ala Val Phe Ser Tyr Phe Cys Asn Leu Val Lys
   145                 150                 155                 160
   Lys Pro Gly Val Glu Phe Pro Phe Pro Asp Ile Tyr Leu Arg Lys Ile
   165 170 175
   Glu Gln Val Lys Leu Gly Glu Met Leu Glu Lys Ser Ala Lys Asp Gln
   180 185 190
   Asp Pro Asp Asp Glu Glu Arg Leu Val Asp Glu Tyr Lys Gln Ile Ala
   195 200 205
   Leu Ile Cys Thr Ser Arg Thr Ile Glu Ala Lys Tyr Ile Asp Phe Leu
   210 215 220
   Leu Glu Leu Ser Asn Leu Lys Val Val Pro Val Gly Ser Pro Val Gln
   225 230 235 240
   Asp Leu Ile Thr Asn Asp Ala Asp Asp Met Glu Leu Ile Asp Trp Leu
   245 250 255
   Gly Ser Lys Asp Glu Asn Ser Thr Val Phe Val Ser Phe Gly Ser Glu
   260 265 270
   Tyr Phe Leu Ser Lys Glu Asp Met Glu Glu Val Ala Leu Gly Leu Glu
   275 280 285
   Leu Ser Asn Val Asn Phe Val Trp Val Ala Arg Phe Pro Lys Gly Glu
   290 295 300
   Glu Gln Asn Leu Glu Asp Ala Leu Pro Lys Gly Phe Leu Glu Arg Ile
   305 310 315 320
   Gly Glu Arg Gly Arg Val Leu Asp Lys Phe Ala Pro Gln Leu Arg Ile
   325 330 335
   Leu Asn His Thr Ser Thr Gly Gly Phe Ile Ser His Cys Gly Trp Asn
   340 345 350
   Ser Val Met Glu Ser Ile His Phe Gly Val Pro Ile Val Ala Met Pro
   355 360 365
   Met His Leu Asp Gln Pro Met Asn Ala Arg Leu Ile Val Glu Leu Gly
   370 375 380
   Val Ala Val Glu Ile Val Arg Asp Asp Asp Gly Lys Ile Tyr Arg Glu
   385 390 395 400
   Glu Ile Ala Lys Thr Leu Lys Asp Val Ile Thr Glu Arg Ile Gly Glu
   405 410 415
   Asn Leu Arg Ala Lys Met Arg Glu Ile Ser Lys Asn Leu Asn Ser Ile
               420                 425                 430
   Ser Gly Glu Glu Met Asp Ala Ala Ala His Glu Leu Ile Gln Phe Cys
           435                 440                 445
   Lys Ile Ser Thr Asn
       450
<210> 2
   <211> 444
   <212> PRT
   <213> Solanum lycopersicon
<400> 2
   Met Glu Ala Lys Lys Asn Lys Met Ser Ile Leu Met Leu Pro Trp Leu
   1               5                   10                  15
   Ala His Gly His Ile Ser Pro Phe Leu Glu Leu Ala Lys Lys Leu Thr
               20                  25                  30
   Asn Arg Asn Phe His Ile Tyr Met Cys Ser Thr Pro Ile Asn Leu Ser
           35                  40                  45
   Ser Ile Lys Lys Asn Ile Thr Lys Lys Tyr Phe Glu Ser Ile Glu Leu
       50                  55                  60
   Val Glu Phe His Leu Pro Ser Leu Pro Asn Leu Pro Pro His Tyr His
   65                  70                  75                  80
   Thr Thr Asn Gly Leu Pro Pro His Leu Met Asn Thr Leu Lys Thr Ala
                   85                  90                  95
   Phe Glu Asn Ala Ser Pro Asn Phe Ser Lys Ile Leu Gln Thr Leu Asn
               100                 105                 110
   Pro Asp Leu Val Ile Tyr Asp Phe Asn Gln Pro Trp Ala Ala Glu Ser
           115                 120                 125
   Ala Ser Ser Val Asn Ile Pro Ala Val Gln Phe Leu Thr Phe Gly Ala
       130                 135                 140
   Ala Val Val Ser Leu Ala Ile His Met Phe Glu Asp Thr Glu Asp Lys
   145                 150                 155                 160
   Phe Pro Phe Pro Glu Ile Tyr Leu His Glu Tyr Glu Met Leu Ser Leu
                   165                 170                 175
   Lys Glu Ala Val Lys Glu Ala Pro Gly Asn Lys Tyr Ser Phe Asp Glu
               180                 185                 190
   Ala Ile Arg Leu Ser Arg Asp Ile Val Leu Val Lys Thr Cys Arg Asp
           195                 200                 205
   Phe Glu Gly Lys Tyr Val Asp Tyr Leu Ser Asn Leu Val Ser Lys Lys
       210                 215                 220
   Ile Val Pro Val Gly Ser Leu Val Gln Glu Ser Ile Ala Arg Asp Asp
   225                 230                 235                 240
   Asn Asp Glu Glu Ile Met Gln Trp Leu Asp Lys Lys Glu Lys Gly Leu
                   245                 250                 255
   Thr Val Phe Val Ser Phe Gly Ser Glu Tyr Phe Leu Ser Lys Glu Asp
               260                 265                 270
   Ile Phe Val Val Ala Arg Gly Leu Glu Leu Ser Lys Val Asn Phe Ile
           275                 280                 285
   Trp Val Ile Arg Phe Ser Gln Gly Glu Arg Ile Ser Ile Gln Asp Ala
       290                 295                 300
   Leu Pro Glu Gly Tyr Leu Glu Arg Val Gly Glu Arg Gly Met Val Ile
   305                 310                 315                 320
   Glu Gly Trp Ala Pro Gln Ala Met Ile Leu Gln His Pro Ser Ile Gly
                   325                 330                 335
   Gly Phe Val Ser His Cys Gly Trp Ser Ser Phe Met Glu Ser Met Lys
               340                 345                 350
   Phe Gly Val Pro Ile Ile Ala Met Pro Met His Ile Asp Gln Pro Met
           355                 360                 365
   Asn Ala Arg Leu Val Glu Tyr Ile Arg Met Gly Val Glu Ala Ala Arg
       370                 375                 380
   Asp Glu Asn Gly Lys Leu Gln Ser Glu Glu Ile Ala Asn Thr Ile Arg
   385                 390                 395                 400
   Lys Val Leu Val Glu Glu Ser Gly Glu Asp Val Arg Asn Lys Ala Lys
                   405                 410                 415
   Glu Leu Ser Gly Lys Met Asn Ala Lys Gly Asp Glu Glu Ile Asp Gly
               420                 425                 430
   Val Val Glu Glu Leu Met Ala Leu Cys Ser Asn Lys
   435 440
<210> 3
   <211> 454
   <212> PRT
   <213> Solanum lycopersicon
<400> 3
   Met Asp Gly Ser Asn Glu Lys Ser Ile Arg Val Leu Met Phe Pro Trp
   1 5 10 15
   Leu Gly His Gly His Ile Ser Pro Phe Phe Glu Leu Ala Lys Lys Leu
   20 25 30
   Val Lys Arg Asn Phe Thr Ile Phe Leu Val Ser Thr Pro Ala Asn Phe
   35 40 45
   Ile Ser Ile Lys Gln Lys Leu Ile His Glu Asn Leu Cys Asp Lys Ile
   50 55 60
   His Leu Phe Asp Leu Arg Leu Pro Ser Leu Pro Asp Leu Pro Pro His
   65 70 75 80
   Tyr His Thr Thr Asn Gly Leu Pro Pro His Leu Met Ser Thr Leu Lys
   85 90 95
   Lys Ala Phe Ala Lys Ser Arg Pro Ile Phe Thr Gln Ile Met Asn Thr
   100 105 110
   Ile Glu Pro Asp Leu Leu Leu Tyr Asp Leu Leu Gln Pro Trp Ala Pro
   115 120 125
   Lys Val Ala Lys Glu Lys Asn Ile Pro Ser Val Val Phe Val Thr Ser
   130 135 140
   Ser Ala Thr Met Phe Ser Tyr Met Phe His Asn Phe Arg Tyr Pro Asn
   145 150 155 160
   Ser Gln Phe Pro Phe Ser Ser Ile Tyr Tyr Arg Asp Tyr Glu Leu Thr
   165 170 175
   Arg Leu Ile Lys Asn Gln Glu Met Glu Thr Ile Glu Gln His Gln Arg
   180 185 190
   Asp Asn Lys Ser Val Lys Met Cys Phe Lys Arg Ser Thr Asn Ile Val
   195 200 205
   Leu Ile Lys Gly Phe Lys Glu Ile Asp Gly Gln Tyr Cys Glu Tyr Ile
       210                 215                 220
   Ser Ser Leu Thr Lys Lys Arg Val Val Pro Val Gly Pro Leu Val Gln
   225                 230                 235                 240
   Glu Gln Thr Ser Glu Asp Asn Asn Ser Gln Ile Leu Thr Trp Leu Asn
                   245                 250                 255
   Gln Lys Ser Lys Gly Ser Thr Ile Phe Val Ser Phe Gly Ser Glu Tyr
               260                 265                 270
   Phe Leu Ser Gln Glu Asp Arg Glu Glu Ile Ala His Gly Leu Glu Gln
           275                 280                 285
   Ser Arg Val Asn Phe Ile Trp Val Val Arg Phe Pro Lys Gly Glu Lys
       290                 295                 300
   Leu Lys Leu Glu Gln Ala Leu Pro Arg Asp Phe Phe Lys Lys Val Gly
   305                 310                 315                 320
   Glu Arg Gly Met Val Val Glu Asp Trp Ala Pro Gln Ala Lys Ile Leu
                   325                 330                 335
   Gly Asn Pro Asn Ile Gly Gly Phe Val Ser His Cys Gly Trp Asn Ser
               340                 345                 350
   Val Leu Glu Ser Met Lys Ile Gly Val Pro Ile Ile Ala Met Pro Met
           355                 360                 365
   His Leu Asp Gln Pro Leu Asn Ala Arg Leu Val Glu Glu Val Gly Ile
       370                 375                 380
   Gly Leu Glu Val Val Arg Asp Lys Asp Gly Lys Leu Asp Gly Glu Gln
   385                 390                 395                 400
   Ile Ser Glu Ile Ile Asn Lys Val Val Leu Glu Lys Glu Gly Glu Ser
                   405                 410                 415
   Ile Arg Glu Lys Ala Lys Lys Met Ser Glu Thr Ile Arg Val Lys Gly
               420                 425                 430
   Asp Glu Glu Ile Asp Asp Val Val Gln Glu Leu Val Asn Leu Cys Lys
           435                 440                 445
   Thr Arg Ser Asn Val Val
       450
<210> 4
   <211> 446
   <212> PRT
   <213> Solanum lycopersicon
<400> 4
   Met Ser Thr Thr Leu Lys Val Leu Met Phe Pro Phe Leu Ala Tyr Gly
   1               5                   10                  15
   His Ile Ser Pro Tyr Leu Asn Val Ala Lys Lys Leu Ala Asp Arg Gly
               20                  25                  30
   Phe Leu Ile Tyr Leu Cys Ser Thr Pro Ile Asn Leu Lys Ser Thr Ile
           35                  40                  45
   Asn Lys Ile Pro Glu Lys Tyr Ala Asp Ser Ile Gln Leu Ile Glu Leu
       50                  55                  60
   His Leu Pro Glu Leu Pro Glu Leu Pro Pro His Tyr His Thr Thr Asn
   65                  70                  75                  80
   Gly Leu Pro Pro Asn Leu Asn His Ile Leu Arg Arg Ala Leu Lys Met
                   85                  90                  95
   Ser Lys Pro Asn Phe Ser Lys Ile Met Gln Asn Leu Lys Pro Asp Leu
               100                 105                 110
   Leu Ile Tyr Asp Ile Leu Gln Gln Trp Ala Glu Asp Val Ala Thr Glu
           115                 120                 125
   Leu Asn Ile Pro Ala Val Lys Leu Leu Thr Ser Gly Val Ala Val Phe
       130                 135                 140
   Ser Tyr Phe Phe Asn Leu Thr Lys Lys Pro Glu Val Glu Phe Pro Tyr
   145                 150                 155                 160
   Pro Ala Ile Tyr Leu Arg Lys Ile Glu Leu Val Arg Trp Cys Glu Thr
                   165                 170                 175
   Leu Ser Lys His Asn Lys Glu Gly Glu Glu His Asp Asp Gly Leu Ala
               180                 185                 190
   Tyr Gly Asn Met Gln Ile Met Leu Met Ser Thr Ser Lys Ile Leu Glu
           195                 200                 205
   Ala Lys Tyr Ile Asp Tyr Cys Ile Glu Leu Thr Asn Trp Lys Val Val
       210                 215                 220
   Pro Val Gly Ser Leu Val Gln Asp Ser Ile Thr Asn Asp Ala Ala Asp
   225                 230                 235                 240
   Asp Asp Met Glu Leu Ile Asp Trp Leu Gly Thr Lys Asp Glu Asn Ser
                   245                 250                 255
   Thr Val Phe Val Ser Phe Gly Ser Glu Tyr Phe Leu Ser Lys Glu Asp
               260                 265                 270
   Val Glu Glu Val Ala Phe Gly Leu Glu Leu Ser Asn Val Asn Phe Ile
           275                 280                 285
   Trp Val Val Arg Phe Pro Lys Gly Glu Glu Lys Asn Leu Glu Asp Val
       290                 295                 300
   Leu Pro Lys Gly Phe Phe Glu Arg Ile Gly Glu Arg Gly Arg Val Leu
   305                 310                 315                 320
   Asp Lys Phe Ala Pro Gln Pro Arg Ile Leu Asn His Pro Ser Thr Gly
                   325                 330                 335
   Gly Phe Ile Ser His Cys Gly Trp Asn Ser Ala Met Glu Ser Ile Asp
               340                 345                 350
   Phe Gly Val Pro Ile Val Ala Met Pro Met Gln Leu Asp Gln Pro Met
           355                 360                 365
   Asn Ala Arg Leu Ile Val Glu Leu Gly Val Ala Val Glu Ile Val Arg
       370                 375                 380
   Asp Asp Asp Gly Lys Ile Tyr Arg Gly Glu Ile Ala Glu Thr Leu Lys
   385                 390                 395                 400
   Gly Val Ile Thr Gly Glu Ile Gly Glu Ile Leu Arg Ala Lys Val Arg
                   405                 410                 415
   Asp Ile Ser Lys Asn Leu Lys Ala Ile Lys Asp Glu Glu Met Asp Val
               420                 425                 430
   Ala Ala Gln Glu Leu Ile Gln Leu Cys Arg Asn Ser Asn Lys
           435                 440                 445
<210> 5
   <211> 473
   <212> PRT
   <213> Stevia rebaudiana
<400> 5
   Met Ala Thr Ser Asp Ser Ile Val Asp Asp Arg Lys Gln Leu His Val
   1               5                   10                  15
   Ala Thr Phe Pro Trp Leu Ala Phe Gly His Ile Leu Pro Tyr Leu Gln
               20                  25                  30
   Leu Ser Lys Leu Ile Ala Glu Lys Gly His Lys Val Ser Phe Leu Ser
           35                  40                  45
   Thr Thr Arg Asn Ile Gln Arg Leu Ser Ser His Ile Ser Pro Leu Ile
       50                  55                  60
   Asn Val Val Gln Leu Thr Leu Pro Arg Val Gln Glu Leu Pro Glu Asp
   65                  70                  75                  80
   Ala Glu Ala Thr Thr Asp Val His Pro Glu Asp Ile Pro Tyr Leu Lys
                   85                  90                  95
   Lys Ala Ser Asp Gly Leu Gln Pro Glu Val Thr Arg Phe Leu Glu Gln
               100                 105                 110
   His Ser Pro Asp Trp Ile Ile Tyr Asp Tyr Thr His Tyr Trp Leu Pro
           115                 120                 125
   Ser Ile Ala Ala Ser Leu Gly Ile Ser Arg Ala His Phe Ser Val Thr
       130                 135                 140
   Thr Pro Trp Ala Ile Ala Tyr Met Gly Pro Ser Ala Asp Ala Met Ile
   145                 150                 155                 160
   Asn Gly Ser Asp Gly Arg Thr Thr Val Glu Asp Leu Thr Thr Pro Pro
                   165                 170                 175
   Lys Trp Phe Pro Phe Pro Thr Lys Val Cys Trp Arg Lys His Asp Leu
               180                 185                 190
   Ala Arg Leu Val Pro Tyr Lys Ala Pro Gly Ile Ser Asp Gly Tyr Arg
           195                 200                 205
   Met Gly Leu Val Leu Lys Gly Ser Asp Cys Leu Leu Ser Lys Cys Tyr
       210                 215                 220
   His Glu Phe Gly Thr Gln Trp Leu Pro Leu Leu Glu Thr Leu His Gln
   225                 230                 235                 240
   Val Pro Val Val Pro Val Gly Leu Leu Pro Pro Glu Ile Pro Gly Asp
                   245                 250                 255
   Glu Lys Asp Glu Thr Trp Val Ser Ile Lys Lys Trp Leu Asp Gly Lys
               260                 265                 270
   Gln Lys Gly Ser Val Val Tyr Val Ala Leu Gly Ser Glu Val Leu Val
           275                 280                 285
   Ser Gln Thr Glu Val Val Glu Leu Ala Leu Gly Leu Glu Leu Ser Gly
       290                 295                 300
   Leu Pro Phe Val Trp Ala Tyr Arg Lys Pro Lys Gly Pro Ala Lys Ser
   305                 310                 315                 320
   Asp Ser Val Glu Leu Pro Asp Gly Phe Val Glu Arg Thr Arg Asp Arg
                   325                 330                 335
   Gly Leu Val Trp Thr Ser Trp Ala Pro Gln Leu Arg Ile Leu Ser His
               340                 345                 350
   Glu Ser Val Cys Gly Phe Leu Thr His Cys Gly Ser Gly Ser Ile Val
           355                 360                 365
   Glu Gly Leu Met Phe Gly His Pro Leu Ile Met Leu Pro Ile Phe Gly
       370                 375                 380
   Asp Gln Pro Leu Asn Ala Arg Leu Leu Glu Asp Lys Gln Val Gly Ile
   385                 390                 395                 400
   Glu Ile Pro Arg Asn Glu Glu Asp Gly Cys Leu Thr Lys Glu Ser Val
                   405                 410                 415
   Ala Arg Ser Leu Arg Ser Val Val Val Glu Lys Glu Gly Glu Ile Tyr
               420                 425                 430
   Lys Ala Asn Ala Arg Glu Leu Ser Lys Ile Tyr Asn Asp Thr Lys Val
           435                 440                 445
   Glu Lys Glu Tyr Val Ser Gln Phe Val Asp Tyr Leu Glu Lys Asn Ala
       450                 455                 460
   Arg Ala Val Ala Ile Asp His Glu Ser
   465                 470
<210> 6
   <211> 481
   <212> PRT
   <213> Stevia rebaudiana
<400> 6
   Met Asp Ala Met Ala Thr Thr Glu Lys Lys Pro His Val Ile Phe Ile
   1               5                   10                  15
   Pro Phe Pro Ala Gln Ser His Ile Lys Ala Met Leu Lys Leu Ala Gln
               20                  25                  30
   Leu Leu His His Lys Gly Leu Gln Ile Thr Phe Val Asn Thr Asp Phe
           35                  40                  45
   Ile His Asn Gln Phe Leu Glu Ser Ser Gly Pro His Cys Leu Asp Gly
       50                  55                  60
   Ala Pro Gly Phe Arg Phe Glu Thr Ile Pro Asp Gly Val Ser His Ser
   65                  70                  75                  80
   Pro Glu Ala Ser Ile Pro Ile Arg Glu Ser Leu Leu Arg Ser Ile Glu
                   85                  90                  95
   Thr Asn Phe Leu Asp Arg Phe Ile Asp Leu Val Thr Lys Leu Pro Asp
               100                 105                 110
   Pro Pro Thr Cys Ile Ile Ser Asp Gly Phe Leu Ser Val Phe Thr Ile
           115                 120                 125
   Asp Ala Ala Lys Lys Leu Gly Ile Pro Val Met Met Tyr Trp Thr Leu
       130                 135                 140
   Ala Ala Cys Gly Phe Met Gly Phe Tyr His Ile His Ser Leu Ile Glu
   145                 150                 155                 160
   Lys Gly Phe Ala Pro Leu Lys Asp Ala Ser Tyr Leu Thr Asn Gly Tyr
                   165                 170                 175
   Leu Asp Thr Val Ile Asp Trp Val Pro Gly Met Glu Gly Ile Arg Leu
               180                 185                 190
   Lys Asp Phe Pro Leu Asp Trp Ser Thr Asp Leu Asn Asp Lys Val Leu
           195                 200                 205
   Met Phe Thr Thr Glu Ala Pro Gln Arg Ser His Lys Val Ser His His
       210                 215                 220
   Ile Phe His Thr Phe Asp Glu Leu Glu Pro Ser Ile Ile Lys Thr Leu
   225                 230                 235                 240
   Ser Leu Arg Tyr Asn His Ile Tyr Thr Ile Gly Pro Leu Gln Leu Leu
                   245                 250                 255
   Leu Asp Gln Ile Pro Glu Glu Lys Lys Gln Thr Gly Ile Thr Ser Leu
               260                 265                 270
   His Gly Tyr Ser Leu Val Lys Glu Glu Pro Glu Cys Phe Gln Trp Leu
           275                 280                 285
   Gln Ser Lys Glu Pro Asn Ser Val Val Tyr Val Asn Phe Gly Ser Thr
       290                 295                 300
   Thr Val Met Ser Leu Glu Asp Met Thr Glu Phe Gly Trp Gly Leu Ala
   305                 310                 315                 320
   Asn Ser Asn His Tyr Phe Leu Trp Ile Ile Arg Ser Asn Leu Val Ile
                   325                 330                 335
   Gly Glu Asn Ala Val Leu Pro Pro Glu Leu Glu Glu His Ile Lys Lys
               340                 345                 350
   Arg Gly Phe Ile Ala Ser Trp Cys Ser Gln Glu Lys Val Leu Lys His
           355                 360                 365
   Pro Ser Val Gly Gly Phe Leu Thr His Cys Gly Trp Gly Ser Thr Ile
       370                 375                 380
   Glu Ser Leu Ser Ala Gly Val Pro Met Ile Cys Trp Pro Tyr Ser Trp
   385                 390                 395                 400
   Asp Gln Leu Thr Asn Cys Arg Tyr Ile Cys Lys Glu Trp Glu Val Gly
                   405                 410                 415
   Leu Glu Met Gly Thr Lys Val Lys Arg Asp Glu Val Lys Arg Leu Val
               420                 425                 430
   Gln Glu Leu Met Gly Glu Gly Gly His Lys Met Arg Asn Lys Ala Lys
           435                 440                 445
   Asp Trp Lys Glu Lys Ala Arg Ile Ala Ile Ala Pro Asn Gly Ser Ser
       450                 455                 460
   Ser Leu Asn Ile Asp Lys Met Val Lys Glu Ile Thr Val Leu Ala Arg
   465                 470                 475                 480
   Asn
<210> 7
   <211> 32
   <212> DNA
   <213> Artificial sequence
<220>
   <223> RT7 forward primer
<400> 7
   attaggatcc aatgggaaca caagtaacag ag 32
<210> 8
   <211> 37
   <212> DNA
   <213> Artificial sequence
<220>
   <223> RT7 reverse primer
<400> 8
   aatactgcag ttaattagta ctaatcttac aaaattg 37
<210> 9
   <211> 37
   <212> DNA
   <213> Artificial sequence
<220>
   <223> RT11 forward primer
<400> 9
   attaggatcc aatggaagcc aagaaaaata aaatgag 37
<210> 10
   <211> 35
   <212> DNA
   <213> Artificial sequence
<220>
   <223> RT11 reverse primer
<400> 10
   aatactgcag tcatttgttg ctgcaaagag ccatc 35
<210> 11
   <211> 34
   <212> DNA
   <213> Artificial sequence
<220>
   <223> RT15 forward primer
<400> 11
   attaggatcc aatggatggt tcgaatgaaa agtc 34
<210> 12
   <211> 35
   <212> DNA
   <213> Artificial sequence
<220>
   <223> RT15 reverse primer
<400> 12
   aatactgcag ctagacaaca tttgatctag tcttg 35
<210> 13
   <211> 38
   <212> DNA
   <213> Artificial sequence
<220>
   <223> RT18 forward primer
<400> 13
   attaggatcc aatgagtact actttaaagg tattgatg **38**
<210> 14
   <211> 36
   <212> DNA
   <213> Artificial sequence
<220>
   <223> RT reverse primer
<400> 14
   aatactgcag attcacttat tactattcct acaaag 36
<210> 15
   <211> 31
   <212> DNA
   <213> Artificial sequence
<220>
   <223> UGT2_la forward primer
<400> 15
   attaggatcc aatggccact tctgactcca t 31
<210> 16
   <211> 32
   <212> DNA
   <213> Artificial sequence
<220>
   <223> UGT2_la reverse primer
<400> 16
   aataaagctt ttagctttcg tggtcaatgg ca 32
<210> 17
   <211> 32
   <212> DNA
   <213> Artificial sequence
<220>
   <223> UGT85C2 forward primer
<400> 17
   attaggatcc aatggacgct atggccacca ct 32
<210> 18
   <211> 32
   <212> DNA
   <213> Artificial sequence
<220>
   <223> UGT85C2 reverse primer
<400> 18
   aataaagctt ttagtttcga gccaagacag tg 32
<210> 19
   <211> 1341
   <212> DNA
   <213> Artificial sequence
<220>
   <223> RT18 open reading frame optimized for expression in Y. lipolitica
<400> 19
   atgtccacca ccctcaaggt cctcatgttc cccttcctcg cttacggcca catctctccc      60
   tacctcaacg ttgccaagaa gctcgccgac cgaggcttcc tcatctacct ctgttccacc     120
   cccatcaacc tcaagtccac catcaacaag atccccgaga agtacgccga ctccatccag     180
   ctcatcgaac tccatctccc cgagcttccc gagctgcctc cccactacca caccaccaac     240
   ggtctgcctc ccaacctcaa ccacatcctc cgacgagccc tcaagatgtc caagcccaac     300
   ttctccaaga tcatgcagaa cctgaagccc gatctgctca tctacgacat tctccagcag     360
   tgggccgagg atgtcgccac cgagcttaac atccccgccg tcaagctgct cacctctggt     420
   gttgctgttt tctcttactt cttcaacctc accaagaagc ccgaggtcga gttcccctac     480
   cccgctatct acctccgaaa gatcgagctg gtccgatggt gcgagactct gtccaagcac     540
   aacaaggaag gtgaggagca cgacgacggc ctcgcctacg gcaacatgca gatcatgctc     600
   atgtccactt ccaagatcct cgaggccaag tacattgact actgcattga gctgaccaac     660
   tggaaggtcg tccccgtcgg ctctctcgtc caggactcca tcaccaacga cgccgctgac     720
   gacgacatgg aactcattga ctggctcggt actaaggacg agaactccac cgtctttgtc     780
   tcttttggct ccgagtactt cctctccaaa gaggacgttg aagaggttgc cttcggtctg     840
   gagctgtcca acgtcaactt catctgggtt gtccgattcc ccaagggtga ggagaagaac     900
   ctcgaggacg ttctgcccaa gggcttcttc gagcgaatcg gtgagcgagg ccgagtcctc     960
   gacaagtttg ctccccagcc ccgaattctc aaccacccct ctaccggtgg tttcatctct    1020
   cactgtggct ggaactccgc catggagtcc attgactttg gtgtccccat tgtcgccatg    1080
   cccatgcagc tcgaccagcc catgaacgcc cgactcattg tcgagcttgg tgttgccgtc    1140
   gagattgtcc gagatgatga tggtaagatc taccgaggtg agattgctga gactctcaag    1200
   ggtgtcatca ccggcgagat tggtgagatc ctccgagcca aggtccgaga catctccaag    1260
   aacctcaagg ccatcaagga cgaggagatg gacgttgctg cccaggagct gatccagctc    1320
   tgccgaaact ccaataaata a                                              1341
<210> 20
   <211> 866
   <212> DNA
   <213> Yarrowia lipolytica
<400> 20
   catgctcact tttgttgtcc tgatgatctc ccgttatttc gccgctcctc tggaaaccat      60
   ccgcccgcaa atcccctctg cccatcttga caatgcacaa tgcatcattc tcagcctgca     120
   tgaatgcgaa agatggcaat attggtggag gaggcgacgg cggtaaacaa tggagataga     180
   aaccacaaaa gaaacctgga aacccaaaat ggactcacga caactccccc actcccccac     240
   tccccatctc cccctgggca tcagttgccc atcggtatct caactgtcgc actagttagc     300
   gcaaccatca catactttag acgccaaaca atgggacaac tcatcgcgcc gaactatggg     360
   cagattttaa ctcgcacaac attaccccaa ctctaaaagg taacctcgac cggaaaacgg     420
   gaagacagga tcagcaaccg tgatcgacag aatcttcagg gcactacagt tgatagacat     480
   aggttatgtt ggtaggtcta gacgggcctc ggggaattga ccccaccagt tgcaagtcac     540
   gtgcccctga tacagctagt ttagcacatc tgcccactac gtctggacgc accatggtgg     600
   tgccagtcgc gtgaactcaa acacccacta gcctcgggaa ggattcagtt aaatccgcac     660
   cttatttcca acacaaagaa gcggttggcg gacaaagaac atgtcctttc tggggcactg     720
   tacattccag gactctgttc aaggtcaaat atacaaaaca cagatagaga aacatagaca     780
   gctgcggcct tataaatacc tgggcgcact tctctctttt tccctcctca tcacacattc     840
   gttcaccact aagtcactcg ttcaaa                                           866
<210> 21
   <211> 999
   <212> DNA
   <213> Yarrowia lipolytica
<400> 21
   ctactgttgc tacgattccc ccattgcaac cacagtttgg ggttaccccg cattatatta      60
   gcatgattac gaaagagata agtatcatat ggaacatgtg aagggtagta tgcaggtccg     120
   gcggagaaag agaatgacgt tttcattaag cgattcgctt ggcggcttgt gggggatgtg     180
   acgatactta cggtaaagac cctgtgtgag agctggtact cgctcgttac ttcgctgatc     240
   tgttgggccg tcaatcgaat ctcgtggaac ttgcattctt cttaactgtg tctatacaag      300
   acacctaatg aaacatacaa gctaccgaaa tcattttact cgtactgacc ggtacggtac      360
   ttgcacaagt agtgaaactt ccgaaaatag ccagcctcat gcatcatcgc ttcacccctt      420
   ctgttgacct caaaagcatt ccaacggtaa aaaattataa cgccgccaac tggatggttg      480
   tgacggcgtt gaccaccaat gtgtgggggc tggcggtagg accgagctta ttcgtcccaa      540
   taagctcttt ggatttgatt ctttggggtg tgtggtaaaa ttcacatggg gaagaacacg      600
   gtggcagttt gaggcagagg cccagcgtgt agttcctagg gcatgaatat accgaactca      660
   tggcgcagaa ttgagctgaa tgcgcaaaaa gctacaggat caaccgcgtt agaaatgccg      720
   caaatgtcca ctaattcccc ggactgttcc aaatgattct gtggggataa atctcaaact      780
   gggttaggct ttgtcacgtt tctttgtgtc gtgtcggttc gtccggggca atgtgcccac      840
   gcttggctgt ctccctacac ctcggtaaaa actatcacat gctgcccctc tcgagcaagc      900
   attaaatgca tatagtcaat ctaacgacat atatataggt agggtgcatc ctccggttta      960
   gctccccaga atatctctta ttcattacac aaaaacaac                             999
<210> 22
   <211> 1000
   <212> DNA
   <213> Yarrowia lipolytica
<400> 22
   gtgcaatcac atgttgctac tgtacctgct gtggaccacg cacggcggaa cgtaccgtac      60
   aaatattttc ttgctcacat gactctctct cggccgcgca cgccggtggc aaattgctct     120
   tgcattggct ctgtctctag acgtccaaac cgtccaaagt ggcagggtga cgtgatgcga     180
   cgcacgaagg agatggcccg gtggcgagga accggacacg gcgagccggc gggaaaaaag     240
   gcggaaaacg aaaagcgaag ggcacaatct gacggtgcgg ctgccaccaa cccaaggagg     300
   ctattttggg tcgctttcca tttcacattc gccctcaatg gccactttgc ggtggtgaac     360
   atggtttctg aaacaacccc ccagaattag agtatattga tgtgtttaag attgggttgc     420
   tatttggcca ttgtggggga gggtagcgac gtggaggaca ttccagggcg aattgagcct     480
   agaaagtggt agcattccaa ccgtctaagt cgtccgaatt gatcgctata actatcacct     540
   ctctcacatg tctacttccc caaccaacat ccccaacctc ccccacacta aagttcacgc     600
   caataatgta ggcactcttt ctgggtgtgg gacagcagag caatacggag gggagattac     660
   acaacgagcc acaattgggg agatggtagc catctcactc gacccgtcga cttttggcaa     720
   cgctcaatta cccaccaaat ttgggctgga gttgagggga ccgtgttcca gcgctgtagg     780
   accagcaaca cacacggtat caacagcaac caacgccccc gctaatgcac ccagtactgc     840
   gcaggtgtgg gccaggtgcg ttccagatgc gagttggcga accctaagcc gacagtgtac     900
   tttttgggac gggcagtagc aatcgtgggc ggaaaccccg gtgtatataa aggggtggag     960
   aggacggatt attagcacca acacacacac ttatactaca                          1000
<210> 23
   <211> 1503
   <212> DNA
   <213> Artificial sequence
<220>
   <223> tHMG optimized for expression in Y. lipolitica
<400> 23
   atgacccagt ctgtgaaggt ggttgagaag cacgttccta tcgtcattga gaagcccagc 60
   gagaaggagg aggacacctc ttctgaagac tccattgagc tgactgtcgg aaagcagccc 120
   aagcccgtga ccgagacccg ttctctggac gacttggagg ctatcatgaa ggcaggtaag 180
   accaagctcc tggaggacca cgaggttgtc aagctctctc tcgaaggcaa gctccctttg 240
   tatgctcttg agaagcagct tggtgacaac acccgagctg ttggcatccg acgatctatc 300
   atctcccagc agtctaatac caagactctt gagacctcaa agctccctta cctgcactac 360
   gactacgacc gtgtttttgg agcctgttgc gagaacgtta ttggttacat gcctctcccc 420
   gttggtgttg ctggccccat gaacattgat ggcaagaact accacattcc tatggccacc 480
   actgagggtt gtcttgttgc ctcaaccatg cgaggttgca aggccatcaa cgccggtggc 540
   ggtgttacca ctgtgcttac tcaggacggt atgacacgag gtccttgtgt ttccttcccc 600
   tctctcaagc gggctggagc cgctaagatc tggcttgatt ccgaggaggg tctcaagtcc 660
   atgcgaaagg ccttcaactc cacctctcga tttgctcgtc tccagtctct tcactctacc 720
   cttgctggta acctgctgtt tattcgattc cgaaccacca ctggtgatgc catgggcatg 780
   aacatgatct ccaagggcgt cgaacactct ctggccgtca tggtcaagga gtacggcttc 840
   cctgatatgg acattgtgtc tgtctcgggt aactactgca ctgacaagaa gcccgcagcg 900
   atcaactgga tcgaaggccg aggcaagagt gttgttgccg aagccaccat ccctgctcac 960
   attgtcaagt ctgttctcaa aagtgaggtt gacgctcttg ttgagctcaa catcagcaag 1020
   aatctgatcg gtagtgccat ggctggctct gtgggaggtt tcaatgcaca cgccgcaaac 1080
   ctggtgaccg ccatctacct tgccactggc caggatcctg ctcagaatgt cgagtcttcc 1140
   aactgcatca cgctgatgag caacgtcgac ggtaacctgc tcatctccgt ttccatgcct 1200
   tctatcgagg tcggtaccat tggtggaggt actattttgg agccccaggg tgctatgctg 1260
   gagatgcttg gcgtgcgagg tcctcacatc gagacccccg gtgccaacgc ccaacagctt 1320
   gctcgcatca ttgcttctgg agttcttgca gcggagcttt cgctgtgttc tgctcttgct 1380
   gccggccatc ttgtgcaaag tcatatgacc cacaaccgtt cccaggctcc tactccggcc 1440
   aagcagtctc aggccgatct gcagcgtctc caaaacggtt cgaatatctg cattcggtca 1500
   tag 1503
<210> 24
   <211> 984
   <212> DNA
   <213> Artificial sequence
<220>
   <223> GGS optimized for expression in Y. lipolitica
<400> 24
   atggattata acagcgcgga tttcaaggag atctggggca aggccgccga caccgcgctg      60
   ctgggaccgt acaactacct cgccaacaac cggggccaca acatcagaga acacttgatc     120
   gcagcgttcg gagcggttat caaggtggac aagagcgatc tcgaaaccat ttcgcacatc     180
   accaagattt tgcataactc gtcgctgctt gttgatgacg tggaagacaa ctcgatgctc     240
   cgacgaggcc tgccggcagc ccattgtctg tttggagtcc cccaaaccat caactccgcc     300
   aactacatgt actttgtggc tctgcaggag gtgctcaagc tcaagtctta tgatgccgtc     360
   tccattttca ccgaggaaat gatcaacttg catagaggtc agggtatgga tctctactgg     420
   agagaaacac tcacttgccc ctcggaagac gagtatctgg agatggtggt gcacaagacc     480
   ggaggactgt ttcggctggc tctgagactt atgctgtcgg tggcatcgaa acaggaggac     540
   catgaaaaga tcaactttga tctcacacac cttaccgaca cactgggagt catttaccag     600
   attctggatg attacctcaa cctgcagtcc acggaattga ccgagaacaa gggattctgc     660
   gaagatatca gcgaaggaaa gttttcgttt ccgctgattc acagcatccg gaccaacccg     720
   gataaccacg agattctcaa cattctcaaa cagcgaacaa gcgacgcttc actcaaaaag     780
   tacgccgtgg actacatgag aacagaaacc aagagtttcg actactgcct caagagaatc     840
   caggccatgt cactcaaggc aagttcgtac attgatgatc tcgcagcagc cggccacgat     900
   gtctccaagt tgcgagccat tttgcattat tttgtgtcca cctctgactg tgaggagaga     960
   aagtactttg aggatgcgca gtga                                            984
<210> 25
   <211> 2232
   <212> DNA
   <213> Artificial sequence
<220>
   <223> CPS from S. rebaudiana optimized for expression in Y. lipolitica
<400> 25
   atgtgcaagg ctgtttccaa ggagtactcc gatctgctcc agaaggacga ggcctctttc      60
   accaagtggg acgacgacaa ggtcaaggac cacctcgaca ccaacaagaa cctctacccc     120
   aacgacgaga tcaaggagtt tgtcgagtcc gtcaaggcca tgttcggctc catgaacgac     180
   ggcgagatta atgtctctgc ttacgacacc gcctgggttg ctctggtcca ggatgtcgac      240
   ggttccggct ctcctcagtt cccttcctct ctcgagtgga tcgccaacaa ccagctgtcc      300
   gacggttctt ggggtgacca cctgctcttc tctgctcacg accgaatcat caacaccctg      360
   gcctgtgtca ttgctctgac ctcttggaac gtccacccct ccaagtgcga gaagggtctg      420
   aacttcctcc gagagaacat ctgcaagctc gaggacgaga acgccgagca catgcccatt      480
   ggcttcgagg tcaccttccc ctctctgatt gacattgcca agaagctcaa cattgaggtc      540
   cccgaggaca cccccgctct caaggagatc tacgctcgac gagacatcaa gctcaccaag      600
   atccccatgg aggttctcca caaggtcccc accactctcc tccactctct cgagggtatg      660
   cccgatctcg agtgggagaa gctgctcaag ctgcagtgca aggacggctc tttcctcttc      720
   tccccctctt ccactgcctt cgccctcatg cagaccaagg acgagaagtg tctccagtac      780
   ctcaccaaca ttgtcaccaa gttcaacggt ggtgtcccca acgtctaccc cgttgacctc      840
   tttgagcaca tctgggttgt tgaccgactc cagcgactcg gtatcgcccg atacttcaag      900
   tccgagatca aggactgtgt cgagtacatc aacaagtact ggaccaagaa cggtatctgc      960
   tgggcccgaa acacccacgt ccaggacatt gacgacaccg ccatgggctt ccgagttctg     1020
   cgagcccacg gctacgatgt cacccccgat gtctttcgac agtttgagaa ggacggcaag     1080
   tttgtctgtt tcgccggtca gtccacccag gccgtcaccg gtatgttcaa cgtctaccga     1140
   gcttctcaga tgctcttccc cggtgagcga atcctcgagg acgccaagaa gttctcctac     1200
   aactacctca aggagaagca gtccaccaac gagctgctcg acaagtggat cattgccaag     1260
   gatctgcccg gtgaggttgg ctacgccctc gacatcccct ggtacgcctc tctgccccga     1320
   ctggagactc gatactacct cgagcagtac ggtggtgagg acgatgtctg gatcggtaag     1380
   accctgtacc gaatgggcta cgtttccaac aacacctacc tcgagatggc caagctcgac     1440
   tacaacaact acgttgccgt cctccagctc gagtggtaca ccatccagca gtggtacgtc     1500
   gacattggta tcgagaagtt cgagtccgac aacatcaagt ccgtccttgt ctcctactac     1560
   ctcgctgctg cctccatctt cgagcccgag cgatccaagg agcgaattgc ctgggccaag     1620
   accaccatcc tcgtcgacaa gatcacctcc atcttcgact cctcccagtc ctccaaggaa     1680
   gatatcaccg ccttcattga caagttccga aacaagtcct cctccaagaa gcactccatc     1740
   aacggcgagc cctggcacga ggtcatggtt gctctcaaga aaactctcca cggctttgcc     1800
   ctcgacgctc tgatgaccca ctctcaggac atccaccccc agctccacca ggcctgggag     1860
   atgtggctca ccaagctcca ggacggtgtt gatgtcactg ctgagctcat ggtccagatg     1920
   atcaacatga ccgccggccg atgggtttcc aaggagctcc tcacccaccc ccagtaccag     1980
   cgactctcca ctgtcaccaa ctctgtctgc cacgacatca ccaagctcca caacttcaag     2040
   gagaactcca ccaccgtcga ctccaaggtc caggagctgg tccagctcgt tttctccgac     2100
   acccccgatg atctcgacca ggacatgaag cagaccttcc tgactgtcat gaaaactttc     2160
   tactacaagg cctggtgcga ccccaacacc atcaacgacc acatctccaa ggtctttgag     2220
   attgtgattt aa                                                         2232
<210> 26
   <211> 2274
   <212> DNA
   <213> Artificial sequence
<220>
   <223> tKS from S. rebaudiana optimized for expression in Y. lipolitica
<400> 26
   atgacctccc acggcggcca gaccaacccc accaacctca tcattgacac caccaaggag      60
   cgaatccaga agcagttcaa gaacgtcgag atctccgttt cctcctacga caccgcctgg     120
   gtcgccatgg tcccctctcc caactccccc aagtctccct gcttccccga gtgtctcaac     180
   tggctcatca acaaccagct caacgacggc tcttggggtc tggtcaacca cacccacaac     240
   cacaaccacc ccctcctcaa ggactctctc tcttccactc tcgcctgcat tgttgctctc     300
   aagcgatgga acgttggcga ggaccagatc aacaagggtc tgtctttcat tgagtccaac     360
   ctcgcctccg ccaccgagaa gtcccagccc tcccccattg gctttgatat catcttcccc     420
   ggtctgctcg agtacgccaa gaacctcgat atcaacctgc tctccaagca gaccgacttc     480
   tctctcatgc tgcacaagcg agagctcgag cagaagcgat gccactccaa cgagatggac     540
   ggctacctgg cctacatttc cgagggtctg ggtaacctct acgactggaa catggtcaag     600
   aagtaccaga tgaagaacgg ttccgttttc aactccccct ctgccaccgc tgctgccttc    660
   atcaaccacc agaaccccgg ctgtctcaac tacctcaact ctctgctcga caagtttggt     720
   aacgccgtcc ccactgtcta cccccacgat ctcttcatcc gactctccat ggtcgacacc     780
   attgagcgac tcggtatttc ccaccacttc cgagtcgaga tcaagaacgt tctcgatgag     840
   acttaccgat gctgggttga gcgagatgag cagatcttca tggacgttgt cacctgtgct     900
   ctggccttcc gactcctccg aatcaacggt tacgaggttt cccccgaccc cctcgccgag     960
   atcaccaacg agctggctct caaggacgag tacgccgccc tcgagactta ccacgcttct    1020
   cacattctgt accaagagga tctgtcctcc ggcaagcaga ttctcaagtc cgccgacttc    1080
   ctcaaggaga tcatctccac tgactccaac cgactctcca agctcatcca caaggaagtc    1140
   gagaacgctc tcaagttccc catcaacacc ggtctggagc gaatcaacac ccgacgaaac    1200
   atccagctct acaacgtcga caacacccga attctcaaga ccacctacca ctcttccaac    1260
   atctccaaca ccgactacct gcgactcgcc gtcgaggact tctacacctg ccagtccatc    1320
   taccgagagg agctcaaggg tctggagcga tgggttgtcg agaacaagct cgaccagctc    1380
   aagtttgccc gacaaaagac tgcctactgc tacttctccg ttgctgccac cctctcttct     1440
   cccgagctct ccgacgcccg aatctcttgg gccaagaacg gtatcctgac cactgttgtc     1500
   gacgacttct ttgacattgg tggcaccatt gacgagctga ccaacctcat ccagtgcgtc     1560
   gagaagtgga acgtcgacgt tgacaaggac tgttgttccg agcacgtccg aatcctcttc     1620
   ctggctctca aggacgccat ctgctggatc ggtgacgagg ccttcaagtg gcaggctcga     1680
   gatgtcactt cccacgtcat ccagacctgg ctcgagctca tgaactccat gctgcgagag     1740
   gccatctgga cccgagatgc ctacgtcccc accctcaacg agtacatgga gaacgcctac     1800
   gtcagctttg ctctcggtcc cattgtcaag cccgccatct actttgtcgg tcccaagctg     1860
   tccgaggaga ttgtcgagtc ctccgagtac cacaacctct tcaagctcat gtccacccag     1920
   ggccgactcc tcaacgatat ccactccttc aagcgagagt tcaaggaagg taagctcaac     1980
   gccgttgctc tgcacctgtc caacggtgag tccggcaagg tcgaggaaga ggtcgtcgag     2040
   gagatgatga tgatgatcaa gaacaagcga aaggagctca tgaagctcat cttcgaggag     2100
   aacggctcca ttgtcccccg agcctgcaag gacgccttct ggaacatgtg ccacgtcctc     2160
   aacttcttct acgccaacga cgacggtttc accggcaaca ccattctcga caccgtcaag     2220
   gacatcatct acaaccctct ggttctggtc aacgagaacg aggagcagag gtaa           2274
<210> 27
   <211> 1578
   <212> DNA
   <213> Artificial sequence
<220>
   <223> KO from Gibberella fujikori optimized for expression in Y. lipolitica
<400> 27
   atgtccaagt ccaactccat gaactccacc tcccacgaga ctctcttcca gcagctcgtt      60
   ctcggcctcg accgaatgcc cctcatggac gtccactggc tcatctacgt tgcctttggt     120
   gcctggctct gctcctacgt catccacgtt ctgtcctctt cctccactgt caaggtcccc     180
   gtcgtcggtt accgatccgt tttcgagccc acctggctcc tccgactgcg attcgtctgg     240
   gagggtggtt ccatcattgg ccagggctac aacaagttca aggactccat cttccaggtc     300
   cgaaagctcg gtaccgacat tgtcatcatc cctcccaact acattgacga ggtccgaaag     360
   ctctcccagg acaagacccg atccgtcgag cccttcatca acgactttgc cggccagtac     420
   acccgaggta tggtctttct gcagtccgat ctccagaacc gagtcatcca gcagcgactc     480
   acccccaagc ttgtctctct caccaaggtc atgaaggaag agctcgacta cgctctgacc     540
   aaggagatgc ccgacatgaa gaacgacgag tgggttgagg tcgacatctc ttccatcatg     600
   gtccgactca tctctcgaat ctccgcccga gttttcctcg gccccgagca ctgccgaaac     660
   caggagtggc tcaccaccac cgccgagtac tccgagtctc tcttcatcac cggcttcatc     720
   ctccgagttg tcccccacat tctccgaccc ttcattgctc ctctgctgcc ctcttaccga     780
   accctgctgc gaaacgtttc ttccggccga cgagtcattg gtgatatcat ccgatcccag     840
   cagggtgacg gtaacgagga catcctctct tggatgcgag atgctgccac tggtgaggag     900
   aagcagatcg acaacattgc ccagcgaatg ctcattctgt ctctcgcctc catccacacc     960
   accgccatga ccatgaccca cgccatgtac gatctgtgtg cctgccccga gtacattgag    1020
   cccctccgag atgaggtcaa gtccgtcgtt ggtgcttctg gctgggacaa gaccgctctc    1080
   aaccgattcc acaagctcga ctctttcctc aaggagtccc agcgattcaa ccccgttttc    1140
   ctgctcacct tcaaccgaat ctaccaccag tccatgaccc tctccgatgg taccaacatc    1200
   ccctccggta cccgaattgc tgtcccctct cacgccatgc tccaggactc cgcccacgtc    1260
   cccggtccca ctcctcccac tgagttcgac ggtttccgat actccaagat ccgatccgac    1320
   tccaactacg cccagaagta cctcttctcc atgaccgact cttccaacat ggcctttggc    1380
   tacggtaagt acgcctgccc cggccgattc tacgcctcca acgagatgaa gctgactctg    1440
   gccattctgc tcctccagtt tgagttcaag ctccccgacg gtaagggccg accccgaaac    1500
   atcaccatcg actccgacat gatccccgac ccccgagctc gactctgtgt ccgaaagcga    1560
   tctctgcgtg acgagtaa                                                  1578
<210> 28
   <211> 1578
   <212> DNA
   <213> Artificial sequence
<220>
   <223> KAH_4 optimized for expression in Y. lipolitica
<400> 28
   atggagtctc tggttgtcca caccgtcaac gccatctggt gcattgtcat tgtcggtatc      60
   ttctccgtcg gctaccacgt ctacggccga gctgttgtcg agcagtggcg aatgcgacga     120
   tctctcaagc tccagggtgt caagggtcct cctccctcca tcttcaacgg taacgtttcc     180
   gagatgcagc gaatccagtc cgaggccaag cactgctccg gtgacaacat catctcccac     240
   gactactctt cttctctgtt cccccacttt gaccactggc gaaagcagta cggccgaatc     300
   tacacctact ccactggcct caagcagcac ctctacatca accaccccga gatggtcaag     360
   gagctctccc agaccaacac cctcaacctc ggccgaatca cccacatcac caagcgactc     420
   aaccccattc tcggtaacgg tatcatcacc tccaacggcc cccactgggc ccaccagcga     480
   cgaatcattg cctacgagtt cacccacgac aagatcaagg gtatggtcgg tctgatggtc     540
   gagtccgcca tgcccatgct caacaagtgg gaggagatgg tcaagcgagg tggtgagatg     600
   ggctgtgaca tccgagtcga cgaggacctc aaggatgtct ccgctgacgt cattgccaag     660
   gcctgtttcg gctcttcctt ctccaagggc aaggccatct tctccatgat ccgagatctg     720
   ctcaccgcca tcaccaagcg atccgtcctc ttccgattca acggtttcac cgacatggtt     780
   ttcggctcca agaagcacgg tgacgttgac attgacgctc tcgagatgga gctcgagtcc     840
   tccatctggg agactgtcaa ggagcgagag attgagtgca aggacaccca caagaaggac     900
   ctcatgcagc tcattctcga gggtgccatg cgatcttgtg acggtaacct gtgggacaag     960
   tctgcttacc gacgattcgt tgtcgacaac tgcaagtcca tctactttgc cggccacgac    1020
   tccaccgccg tttccgtttc ttggtgcctc atgctgctcg ctctcaaccc ctcttggcag    1080
   gtcaagatcc gagatgagat tctgtcctcc tgcaagaacg gtatccccga cgccgagtcc    1140
   atccccaacc tcaagaccgt caccatggtc atccaggaga ctatgcgact ctaccctccc    1200
   gctcccattg tcggccgaga ggcctccaag gacattcgac tcggtgatct ggttgtcccc    1260
   aagggtgtct gtatctggac cctcatcccc gctctgcacc gagatcccga gatctggggt    1320
   cccgacgcca acgacttcaa gcccgagcga ttctccgagg gtatctccaa ggcctgcaag    1380
   tacccccagt cctacatccc ctttggcctc ggcccccgaa cctgtgtcgg caagaacttt    1440
   ggtatgatgg aggtcaaggt cctcgtttct ctgattgtct ccaagttctc cttcactctg    1500
   tctcccacct accagcactc tccctcccac aagctgctcg tcgagcccca gcacggtgtt    1560
   gtcatccgag ttgtataa                                                  1578
<210> 29
   <211> 2133
   <212> DNA
   <213> Artificial sequence
<220>
   <223> CPR_optimized for expression in Y. lipolitica
<400> 29
   atgtcctcct cttcttcttc ttccacctcc atgattgatc tcatggctgc catcatcaag      60
   ggtgagcccg tcattgtctc cgaccccgcc aacgcctccg cctacgagtc cgttgctgcc     120
   gagctgtcct ccatgctcat cgagaaccga cagtttgcca tgatcgtcac cacctccatt     180
   gctgttctca ttggctgcat tgtcatgctc gtctggcgac gatctggctc cggtaactcc     240
   aagcgagtcg agcccctcaa gcccctggtc atcaagcccc gagaagagga gatcgacgac     300
   ggccgaaaga aggtcaccat cttctttggc acccagaccg gtactgctga gggcttcgcc     360
   aaggctctcg gtgaggaagc caaggctcga tacgaaaaga cccgattcaa gattgtcgac     420
   ctcgatgatt acgctgccga tgacgacgag tacgaggaga agctcaagaa agaggacgtt     480
   gccttcttct tcctcgccac ctacggtgac ggtgagccca ccgacaacgc tgcccgattc     540
   tacaagtggt tcaccgaggg taacgaccga ggcgagtggc tcaagaacct caagtacggt     600
   gttttcggtc tgggcaaccg acagtacgag cacttcaaca aggttgccaa ggttgtcgac      660
   gacatcctcg tcgagcaggg tgcccagcga ctcgtccagg tcggcctcgg tgatgatgac      720
   cagtgcatcg aggacgactt cactgcctgg cgagaggctc tgtggcccga gctcgacacc      780
   attctgcgag aggaaggtga caccgccgtt gccaccccct acaccgccgc cgtcctcgag      840
   taccgagtct ccatccacga ctccgaggat gccaagttca acgacatcaa catggccaac      900
   ggtaacggct acaccgtctt tgacgcccag cacccctaca aggccaacgt cgccgtcaag      960
   cgagagctcc acacccccga gtccgaccga tcttgtatcc acctcgagtt tgacattgct     1020
   ggttccggtc tgacctacga gactggtgac cacgttggtg tcctctgtga caacctgtcc     1080
   gagactgtcg acgaggctct gcgactcctc gacatgtccc ccgacactta cttctctctg     1140
   cacgccgaga aagaggacgg tactcccatc tcttcttctc tgccccctcc cttccctccc     1200
   tgcaacctgc gaaccgctct gacccgatac gcctgcctcc tctcttctcc caagaagtct     1260
   gctctcgttg ctctggccgc ccacgcctcc gaccccaccg aggctgagcg actcaagcac     1320
   ctcgcctctc ccgctggcaa ggacgagtac tccaagtggg ttgtcgagtc ccagcgatct     1380
   ctgctcgagg tcatggccga gttcccctcc gccaagcccc ctctcggtgt tttcttcgcc     1440
   ggtgttgctc cccgactcca gccccgattc tactccatct cctcttcccc caagatcgcc     1500
   gagactcgaa tccacgttac ctgtgctctg gtctacgaga agatgcccac cggccgaatc     1560
   cacaagggtg tctgctccac ctggatgaag aacgccgttc cctacgagaa gtccgagaac     1620
   tgttcctctg ctcccatctt tgtccgacag tccaacttca agctcccctc cgactccaag     1680
   gtccccatca tcatgattgg ccccggtacc ggcctcgccc ccttccgagg cttcctgcag     1740
   gagcgactcg ccctcgtcga gtccggtgtc gagctcggcc cctccgtcct cttctttggc     1800
   tgccgaaacc gacgaatgga cttcatctac gaagaggagc tccagcgatt cgtcgagtcc     1860
   ggtgctctcg ccgagctctc cgttgccttc tcccgagagg gtcccaccaa ggagtacgtc     1920
   cagcacaaga tgatggacaa ggcctccgac atctggaaca tgatctccca gggcgcctac     1980
   ctctacgtct gcggtgacgc caagggtatg gcccgagatg tccaccgatc tctgcacacc     2040
   attgcccagg agcagggctc catggactcc accaaggccg agggtttcgt caagaacctc     2100
   cagacctccg gccgatacct ccgagatgtc tgg                                  2133
<210> 30
   <211> 1446
   <212> DNA
   <213> Artificial sequence
<220>
   <223> UGT1 optimized for expression in Y. lipolitica
<400> 30
   atggacgcca tggccaccac cgagaagaag ccccacgtca tcttcatccc cttccccgcc      60
   cagtcccaca tcaaggccat gctcaagctc gcccagctcc tccaccacaa gggcctccag     120
   atcacctttg tcaacaccga cttcatccac aaccagttcc tcgagtcctc cggcccccac     180
   tgtctggacg gtgctcccgg tttccgattt gagactatcc ccgatggtgt ctcccactcc     240
   cccgaggcct ccatccccat ccgagagtct ctgctccgat ccattgagac taacttcctc     300
   gaccgattca ttgatctcgt caccaagctc cccgatcctc ccacctgtat catctccgac     360
   ggtttcctgt ccgttttcac cattgatgct gccaagaagc tcggtatccc cgtcatgatg     420
   tactggactc tggctgcctg tggtttcatg ggtttctacc acatccactc tctgatcgag     480
   aagggctttg ctcctctcaa ggacgcctcc tacctcacca acggttacct cgacaccgtc     540
   attgactggg tccccggtat ggagggtatc cgactcaagg acttccccct cgactggtcc     600
   accgacctca acgacaaggt tctcatgttc accaccgagg ctccccagcg atcccacaag     660
   gtttcccacc acatcttcca caccttcgac gagctcgagc cctccatcat caagactctg     720
   tctctgcgat acaaccacat ctacaccatt ggccccctcc agctcctcct cgaccagatc     780
   cccgaggaga agaagcagac cggtatcacc tctctgcacg gctactctct cgtcaaggaa     840
   gagcccgagt gcttccagtg gctccagtcc aaggagccca actccgttgt ctacgtcaac     900
   tttggctcca ccaccgtcat gtctctcgag gacatgaccg agtttggctg gggtctggcc      960
   aactccaacc actacttcct gtggatcatc cgatccaacc tcgtcattgg cgagaacgcc    1020
   gttctgcctc ccgagctcga ggagcacatc aagaagcgag gcttcattgc ctcttggtgc    1080
   tcccaggaga aggttctcaa gcacccctcc gtcggtggtt tcctgaccca ctgcggctgg    1140
   ggctccacca ttgagtctct gtccgctggt gtccccatga tctgctggcc ctactcctgg    1200
   gaccagctca ccaactgccg atacatctgc aaggagtggg aggttggtct ggagatgggt    1260
   accaaggtca agcgagatga ggtcaagcga ctcgtccagg agctcatggg cgagggtggt    1320
   cacaagatgc gaaacaaggc caaggactgg aaggagaagg cccgaattgc cattgccccc    1380
   aacggctctt cttctctcaa cattgacaag atggtcaagg agatcactgt tctcgctcga    1440
   aactaa                                                               1446
<210> 31
   <211> 1419
   <212> DNA
   <213> Artificial sequence
<220>
   <223> UGT2 variant optimized for expression in Y. lipolitica
<400> 31
   atggccacct ccgactccat tgttgacgac cgaaagaagc tccacattgt catgttcccc      60
   tggctcgcct ttggccacat catcccctat ctcgagcttt ccaagctcat tgcccagaag     120
   ggccacaagg tttccttcct ctccaccacc aagaacattg accgactctc ctcccacatc     180
   tctcccctca tcaactttgt caagctcacc ctcccccgag tccaggagct gcccgaggac     240
   gccgaggcca ccactgatgt ccaccccgag gatatcccct acctcaagaa ggcctccgac     300
   ggcctccagc ccgaggtcac tgagttcctc gagcagcact ctcccgactg gatcatctac     360
   gactacaccc actactggct ccccgagatt gccaagtctc tcggtgtctc tcgagcccac     420
   ttctccgtca ccaccccctg ggccattgct tacatgggtc ccactgccga tgccatgatc     480
   aacggttccg actaccgaac cgagcttgag gacttcaccg tccctcccaa gtggttcccc     540
   ttccccacca ccgtctgctg gcgaaagcac gatctggccc gactcgtccc ctacaaggct     600
   cccggtatct ccgacggtta ccgaatgggc ctcgtcatca agggctgcga ctgtctgctc     660
   tccaagacct accacgagtt cggtactcag tggctccgac ttctcgagga gctgcaccga     720
   gtccccgtca tccccgttgg tctgctccct ccctccatcc ccggctctga caaggacgac     780
   tcttgggttt ccatcaagga gtggctcgac ggccaggaga agggctccgt tgtctacgtt     840
   gctctcggtt ccgaggttct cgtcacccag gaagaggttg tcgagcttgc tcacggtctg     900
   gagctgtccg gtctgccctt cttctgggcc taccgaaagc ccaagggtcc cgccaagtcc     960
   gactccgtcg agcttcccga tggtttcgtc gagcgagtcc gagatcgagg tctggtctgg    1020
   acctcttggg ctccccagct ccgaatcctc tcccacgagt ccgttgctgg tttcctcacc    1080
   cactgcggtt ccggctccat tgtcgagggc ctcatgttcg gccaccctct catcatgctc    1140
   cccatcttcg gtgaccagcc cctcaacgcc cgactccttg aggacaagca ggtcggtatc    1200
   gagatccccc gaaacgagga agatggttct ttcacccgag actctgttgc cgagtctctg    1260
   cgactcgtca tggtcgagga agagggtaag atctaccgag agaaggccaa ggagatgtcc    1320
   aagctctttg gcgacaagga cctccaggac cagtacgtcg acgactttgt cgagtacctc    1380
   cagaagcacc gacgagctgt tgccattgac cacgaaagc                           1419
<210> 32
   <211> 1383
   <212> DNA
   <213> Artificial sequence
<220>
   <223> UGT3 optimized for expression in Y. lipolitica
<400> 32
   atggccgagc agcagaagat caagaagtct ccccacgttc tgctcatccc cttccctctg      60
   cagggccaca tcaacccctt catccagttc ggcaagcgac tcatctccaa gggtgtcaag     120
   accactctgg tcaccaccat ccacaccctc aactccactc tcaaccactc caacaccacc     180
   accacctcca tcgagatcca ggccatctcc gacggctgtg acgagggtgg tttcatgtct     240
   gctggtgagt cttacctcga gactttcaag caggtcggtt ccaagtctct ggctgacctc     300
   atcaagaagc tccagtccga gggtaccacc attgacgcca tcatctacga ctccatgacc     360
   gagtgggttc tcgatgtcgc catcgagttt ggtattgacg gtggctcctt cttcacccag     420
   gcctgtgtcg tcaactctct ctactaccac gtccacaagg gtctgatctc tctgcccctc     480
   ggcgagactg tctccgtccc cggtttcccc gttctgcagc gatgggagac tcctctcatt     540
   ctccagaacc acgagcagat ccagtccccc tggtcccaga tgctcttcgg ccagttcgcc     600
   aacattgacc aggcccgatg ggttttcacc aactccttct acaagctcga ggaagaggtc     660
   attgagtgga cccgaaagat ctggaacctc aaggtcattg gccccaccct cccctccatg     720
   tacctcgaca agcgactcga tgacgacaag gacaacggtt tcaacctcta caaggccaac     780
   caccacgagt gcatgaactg gctcgacgac aagcccaagg agtccgttgt ctacgttgcc     840
   tttggctctc tggtcaagca cggccccgag caggttgagg agatcacccg agctctgatt     900
   gactccgatg tcaacttcct gtgggtcatc aagcacaagg aagagggtaa gctccccgag     960
   aacctgtccg aggtcatcaa gaccggcaag ggcctcattg ttgcctggtg caagcagctc    1020
   gacgttctcg cccacgagtc cgtcggctgc tttgtcaccc actgcggttt caactccacc    1080
   ctcgaggcta tctctctcgg tgtccccgtt gttgccatgc cccagttctc cgaccagacc    1140
   accaacgcca agctcctcga tgagattctc ggtgtcggtg tccgagtcaa ggctgacgag    1200
   aacggtattg tccgacgagg taacctggct tcttgtatca agatgatcat ggaggaagag    1260
   cgaggtgtca tcatccgaaa gaacgccgtc aagtggaagg atctggccaa ggttgctgtc    1320
   cacgagggtg gctcttccga caacgacatt gtcgagtttg tctccgagct catcaaggcc    1380
   taa                                                                  1383
<210> 33
   <211> 1377
   <212> DNA
   <213> Artificial sequence
<220>
   <223> UGT4 optimized for expression in Y. lipolitica
<400> 33
   atggagaaca agaccgagac taccgtccga cgacgacgac gaatcattct cttccccgtc      60
   cccttccagg gccacatcaa ccccattctg cagctcgcca acgttctgta ctccaagggc     120
   ttctccatca ccatcttcca caccaacttc aacaagccca agacctccaa ctacccccac     180
   ttcactttcc gattcatcct cgacaacgac ccccaggacg agcgaatctc caacctgccc     240
   acccacggtc ctctggctgg tatgcgaatc cccatcatca acgagcacgg tgctgacgag     300
   ctccgacgag agctcgagct gctcatgctc gcctccgaag aggacgagga agtctcctgt     360
   ctgatcaccg atgctctgtg gtactttgcc cagtccgtcg ccgactctct caacctgcga     420
   cgactcgttc tcatgacctc ctctctgttc aacttccacg cccacgtttc tctgccccag     480
   tttgacgagc tcggttacct cgaccccgat gacaagaccc gactcgagga gcaggcttcc     540
   ggtttcccca tgctcaaggt caaggacatc aagtccgcct actccaactg gcagattctc     600
   aaggagattc tcggcaagat gatcaagcag accaaggcct cctccggtgt catctggaac     660
   tccttcaagg agctcgagga gtccgagctc gagactgtca tccgagagat ccccgctccc     720
   tctttcctca tccccctgcc caagcacctc accgcttcct cctcttctct gctcgaccac     780
   gaccgaaccg tctttcagtg gctcgaccag cagccccctt cctccgtcct ctacgtttcc     840
   ttcggctcca cctccgaggt cgacgagaag gacttcctcg agattgctcg aggcctcgtt     900
   gactccaagc agtccttcct gtgggttgtc cgacccggct ttgtcaaggg ctccacctgg     960
   gttgagcccc tgcccgatgg tttcctcggt gagcgaggcc gaattgtcaa gtgggtcccc    1020
   cagcaggaag ttctggccca cggtgccatt ggtgccttct ggacccactc cggctggaac    1080
   tccactctcg agtccgtctg cgagggtgtc cccatgatct tctccgactt tggcctcgac    1140
   cagcccctca acgcccgata catgtccgat gttctcaagg tcggtgtcta cctcgagaac    1200
   ggctgggagc gaggtgagat tgccaacgcc atccgacgag tcatggtcga cgaggaaggt    1260
   gagtacatcc gacagaacgc ccgagtcctc aagcagaagg ccgatgtctc tctcatgaag    1320
   ggtggttctt cttacgagtc tctcgagtct ctcgtttcct acatctcttc tttgtaa       1377
<210> 34
   <211> 1419
   <212> DNA
   <213> Artificial sequence
<220>
   <223> UGT2 variant optimized for expression in Y. lipolitica
<400> 34
   atggctactt ccgactccat tgtcgacgac cgaaagaagc tccacattgt catgttcccc      60
   tggctcgcct ttggccacat cattccctac ctcgagcttt ccaagctcat tgcccagaag     120
   ggccacaagg tttctttcct ctccaccacc aagaacattg accgactctc ctcccacatc     180
   tctcctctca tcaacgttgt ccagctcacc ctcccccgag tccaggagct gcccgaggac     240
   gccgaggcca ccaccgatgt ccaccccgag gatatcccct acctcaagaa ggcctccgac     300
   ggtctgcagc ccgaggtcac cgagttcctc gagcagcact ctcccgactg gatcatctac     360
   gactacaccc actactggct cccctccatt gccaccaagc acggtgtctc tcgagcccac     420
   ttctccgtca ccaccccctg ggccattgcc tacatgggcc ccactgctga cgccatgatc     480
   aacggttccg atggccgaac cacccccgag gacttcactg tccctcccaa gtggttcccc     540
   ttccccacca aggtctgctg gcgaaagcac gatctggccc gactcgttcc ctacaaggcc     600
   cccggtatct ccgacggcta ccgaatgggt ctggtcatca agggctgcga ctgtctgctc     660
   tccaagacct accacgagtt tggcacccag tggctccgac tcctcgagac tctccaccga     720
   aagcccgtca tccccgtcgg tctgctccct ccctccatcc ccggctccga caaggacgac     780
   tcttgggttt ccatcaagga gtggctcgac ggccaggaga agggctctgt tgtctacgtt     840
   gctctcggtt ccgaggttct cgtcacccag gacgaggttg ttgagctggc ccacggtctg     900
   gagctgtccg gcctcccctt cgtctgggct taccgaaacc ccaagggtcc cgccaagtcc     960
   gactccgtcg agcttcccga tggtttcgtc gagcgagtcc gagatcgagg tctggtctgg    1020
   acctcttggg ctccccagct ccgaatcctc tcccacgagt ccgtctgtgg tttcctcacc    1080
   cactgcggtt ccggctccat cgtcgagggt ctgatgttcg gccaccccct catcatgctc    1140
   cccatcttcg gtgaccagcc cctcaacgcc cgactccttg aggacaagca ggtcggtatc    1200
   gagatccccc gaaacgaaga ggacggttcc ttcacccgag actctgttgc tgagtctctc    1260
   cgactcgtca tggtcgagga agagggtaag atctaccgag agaaggccaa ggagatgtcc    1320
   aagctgttcg gtgacaagga tctccaggac cagtacgtcg acgactttgt cgagtacctc    1380
   cagaagcacc gacgagctgt tgccattgac cacgagtct                           1419